# EUROPEAN PATENT APPLICATION

(11) **EP 1 262 553 A1**
(43) Date of publication of application: **04.12.2002**
(21) Application number: 01401338.7
(22) Date of filing: 22.05.2001
(51) Int. Cl.: C12N 15/62, C12Q 1/66, C12N 9/02, G01N 33/533

(54) **Luciferase-selection marker fusion proteins, polynucleotides encoding such proteins, and uses thereof**

(71) Applicant: ADEREGEM (Association pour le Développement de la Recherche en Génétique Moléculaire, F-75012 Paris (FR)
(72) Inventor: Angrand, Pierre-Olivier, 67000 Strasbourg (FR)
(74) Representative: Warcoin, Jacques

(57) **Abstract**

The invention relates to new active recombinant fusion proteins comprising a *luciferase,* or a fragment, or a variant thereof fused to a selection marker protein, or a fragment or a variant thereof, said fusion proteins having at least a light-producing activity and a selection marker activity. The corresponding polynucleotides, DNA cassettes, vectors and kits are claimed. The invention also relates to a method of selecting cells into the genome of which a genetic element such as a tag has been targeted into at least one predefined locus, and a method for trapping unknown genes.

## Description

The present invention relates to the technical field of genetic manipulation, functional genomics, and more specifically, to the use of a new vector system to facilitate the identification and functional characterization of targeted genes. More precisely, the invention relates to new active recombinant fusion proteins comprising a *luciferase*, or a fragment, or a variant thereof fused to a selection marker protein, or a fragment or a variant thereof, said fusion proteins having at least a light-producing activity and a selection marker activity. The corresponding polynucleotides, DNA cassettes, vectors and kits are claimed. The invention also relates to a method of selecting cells into the genome of which a genetic element has been targeted into at least one predefined locus, and a method for trapping unknown genes.

Gene targeting is the only procedure that can produce defined modifications at a given locus in the genome of eukaryotic cells. Therefore, gene targeting is an attractive approach to functional genomics, to gene therapy as well as to genome alteration for nuclear transfer-based modifications in livestock.

Gene targeting is a complex exercise in somatic cells due to the fact that the absolute frequency of homologous recombination in those cells is two orders of magnitude lower than in embryonic stem (ES) cells (Arbones *et al.*, 1994 ; Hanson *et al*., 1995 ; Brown *et al.*, 1997 ; Yañez *et al.*, 1998 ; Sedivy *et al.*, 1999). In addition, frequencies of nonhomologous recombination are typically very high in somatic cells (Waldman, 1992). In mammalian somatic cells, the great majority of stable transformants are the result of nonhomologous random recombination (NHR) of the construct into the genome, and gene targeting (GT) by homologous recombination is orders of magnitude less efficient. The low ratios of gene targeting to nonhomologous recombination (GT:NHR), in the range of 1:30 - 1:40 000 (Ya 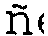 ez *et al.*, 1998 ; Sedivy *et al.*, 1999), led to the development of genetic enrichment strategies to prevent nonhomologous recombinants from scoring as colonies. Two fundamentally different enrichment procedures have been developed. The first one is, in genetic terms, negative and selects against recombination at non-homologous *loci* (Mansour *et al.*, 1988). This method, referred to as positive-negative selection, relies on the use of a negatively selectable gene that is placed on the flanks of the targeting vector. The second approach is, in genetic terms, positive and selects for recombination at the homologous locus (Jasin *et al.*, 1988 ; Sedivy *et al.*, 1989). This method, referred to as promoterless selection, relies on the use of a positively selectable gene whose expression is made conditional on recombination at the homologous target site. While positive-negative selection vectors achieve enrichments of only 2-9 fold, promoterless selection vectors typically achieve enrichments of 500-5000 fold.

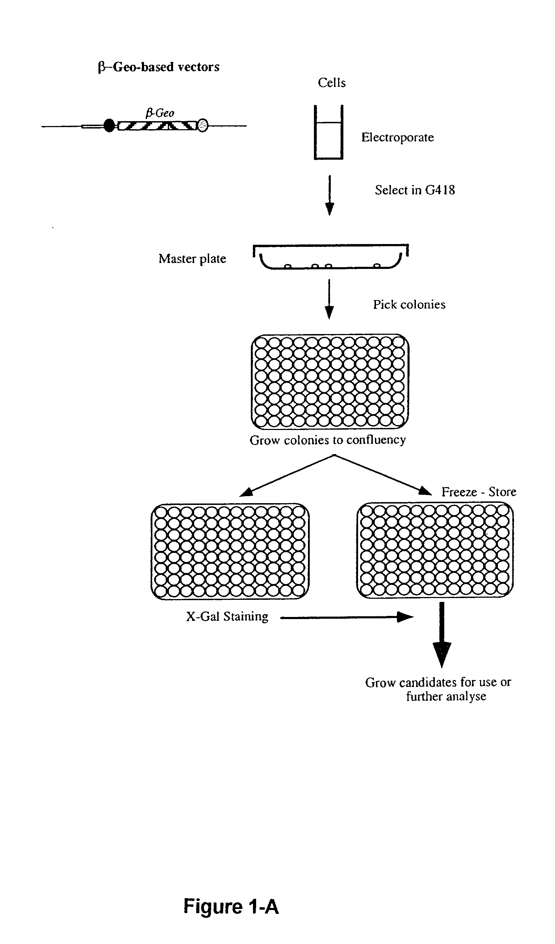

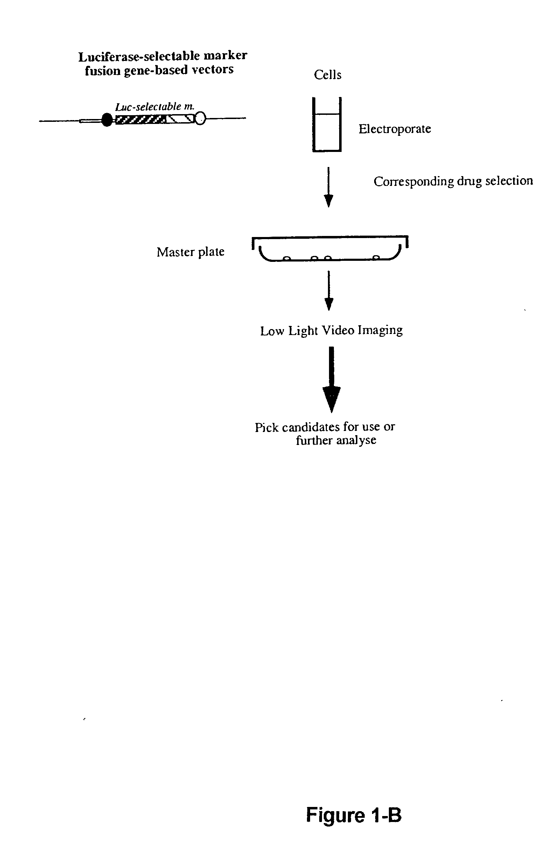

In several cases, the addition of a reporter component proved to be useful for the screening and characterization of homologous recombinants (see for example Zambrowicz *et al.,* 1998; Wiles *et al.,* 2000). For such applications, the most popular marker is the β-Geo gene. The β-Geo gene results from the fusion of lacZ and neo genes, conferring G418 resistance (neomycin phosphotransferase activity) and (β-galactosidase activity (Friedrich *et al.,* 1991). However, assays for β-galactosidase activity are performed by X-GAL histochemistry in replica plated colonies after fixation and permeabilization of cells (Figure 1A). Thus, assays for β-galactosidase activity which are performed on dead cells are time consuming.

These observations lead to the conclusion that nowadays one cannot easily and rapidly perform selection and screening of positive clones that have undergone homologous recombination. There is a need to provide a vector system that includes specifically designed sequences for easy selection and much faster screening of positive recombinant lines compared to the other available systems.

Thus, the problem to be solved is to develop new marker genes that will circumvent all the drawbacks of the existing reporter systems.

The inventor has solved the problem underlying the invention by developing a novel system based on the properties of fusion genes between the *luciferase* cDNAs and selectable marker genes. In contrast to the *β-Geo* gene, one virtue of these new fusion genes is that the *luciferase* activities may be monitored on master dishes in living conditions by low light video imaging (see for example, Langridge *et al.*, 1996; Lorenz *et al.*, 1996 and Figure 1B), and therefore provide a much faster tool for the screening of positive recombinant lines. Another advantage of the system of the invention is that the multiplicity of *luciferase* genes available allows one to catalyse several specific substrates that emit lights of different colors. Consequently the activity of several luciferases can be discriminated in the same cell allowing a gain of time. Another advantage of the system of the invention is the greater sensibility over prior art fusion proteins using autofluorescent proteins such as green fluorescent protein (GFP); indeed, the *luciferase* enzymatic activity allows one to accumulate light signals.

The invention provides an active recombinant fusion protein comprising a *luciferase,* or a fragment, or a variant thereof fused to a selection marker protein, or a fragment or a variant thereof, said fusion protein having at least a light-producing activity and a selection marker activity.

By "active" fusion protein, it is meant that said protein is able to have an enzymatic *luciferase* activity and a selection marker activity in an appropriate reaction mix or in an appropriate host cell. In a preferred embodiment such fusion protein is soluble in aqueous medium.

By « light producing-activity » it is meant that said *luciferase* fusion protein is detectable by photon emission or produces a photon emitting detectable product. The photon emission is generated either by luminescence, fluorescence or phosphorescence. According to a preferred embodiment, the photon emission is generated by luminescence, more precisely by bioluminescence. As used herein, luminescence refers to the detectable electromagnetic radiation, generally, UV, IR or visible EM (EM:electromagnetic) radiation that is produced when the excited product of an exergonic chemical process reverts to its ground state with the emission of light. Bioluminescence is chemiluminescence that results from a chemical reaction using biological molecules (or synthetic versions or analogs thereof) as substrates and/or enzymes.

By "luciferases", it is meant all proteins that catalyze or initiate the bioluminescent reaction. Luciferases refer to any compound that, in the presence of any necessary activators, catalyze the oxidation of a bioluminescence substrate called "luciferin" in the presence of molecular oxygen, whether free or bound, from a lower energy state to a higher energy state such that the substrate, upon return to the lower energy state, emits light. *Luciferase* refers to oxygenases that catalyze a light emitting reaction. For purposes herein, *luciferase* is broadly used to encompass (i) enzymes that act catalytically to generate light by oxidation of a substrate, such as *photinus* and *Renilla* luciferases (Wilson and Hastings, 1998) and also (ii) photoproteins, such as *æquorin* and *Obelin*, that act, though not strictly catalytically (since such proteins are exhausted in the reaction), in conjunction with a substrate in the presence of oxygen to generate light. These luciferases, including photoproteins, such as *æquorin*, are herein also included among the luciferases. The luciferases, such as *photinus* and *Renilla* luciferases, are enzymes that act catalytically and that are unchanged during the bioluminescence generating reaction. The *luciferase* photoproteins, such as the *aequorin* and *obelin* photoproteins to which luciferin is non-covalently bound, are changed, such as by release of the luciferin, during the bioluminescence generating reaction.

By "*luciferase* fragment", it is meant any biologically active fragment of *luciferase*, that is to say any fragment of a natural or variant *luciferase* that retains the ability to catalyze the bioluminescent reaction. A man skilled in the art knows how to demonstrate such *luciferase* enzymatic activity, by, for example, measuring the light emitted by the catalysis of a luciferin substrate by the *luciferase* fragment.

By "*luciferase* variant" or "selection marker variant", it is meant all the wild type luciferases, respectively all the wild type selection marker proteins or fragments thereof, that are naturally existing and which are corresponding to truncations, substitutions, deletions and/or additions of amino-acid moiety(ies). By "*luciferase* variant" or "selection marker variant" it is also meant all the synthetic variants for which the above modifications are not naturally occurring but have been artificially introduced, by genetic engineering for example; such variants produced for example by mutagenesis can have one or more properties, such as thermal or pH stability, or longer or shorter half-life, that differ from the naturally-occurring protein.

Said *luciferase* of the invention can be isolated from numerous organisms and sources of bioluminescence generating systems; some representative genera and species that exhibit bioluminescence are set forth in the following table 1 (reproduced in part from Hastings, 1995; see also US 6,152,358).

**Table 1 :**

| **Representative luminous organisms** | |
|---|---|
| Type of organism | Representative genera |
| Bacteria | *Photobacterium* |
| | *Vibrio* |
| | *Xenorhabdus* |
| Mushrooms | *Panus, Armillaria* |
| | *Pleurotus* |
| Dinoflagellates | *Gonyaulax* |
| | *Pyrocustis* |
| | *Noctiluca* |
| Cnidaria (coelenterates) | |
| Jellyfish | *Aequorea* |
| Hydroid | *Obelia* |
| Sea Pansy | *Renilla* |
| Ctenophores | *Mnemiopsis* |
| | *Beroe* |
| Annelids | |
| Earthworms | *Diplocardia* |
| Marine polychætes | *Chætopterus, Phyxotrix* |
| Syllid fireworm olliscs | *Odontosyllis* |
| Limpet | *Latia* |
| Clam | *Pholas* |
| Squid | *Heterotheuthis* |
| | *Heterocarpus* |
| Crustacea | |
| Ostracod | *Vargula* (*Cypridina*) |
| Shrimp (euphausids) | |
| | Menanyctiphanes |
| | *Acanthophyra* |
| | *Oplophorus* |

| Type of organism | Representative genera |
|---|---|
| | *Gnathophausia* |
| Decapod | *Sergestes* |
| Copepods | |
| Insects | |
| Coleopterids (beetles) | |
| Firefly | *Photinus, Photuris* |
| Click beetles | *Pyrophorus* |
| Railroad worm | *Phengodes, Phrixothrix* |
| Diptera (flies) | *Arachnocampa* |
| Echinoderms | |
| Brittle stars | *Ophiopsila* |
| Sea cucumbers | *Lætmogone* |
| Chordates | |
| Tunicates | *Pyrosoma* |
| Fish | |
| Cartilainous | *Squalus* |
| Bony | |
| Ponyfish | *Leiognathus* |
| Flashlight fish | *Photoblepharon* |
| Angler fish | *Cryptopsaras* |
| Midshipman | *Porichthys* |
| Lantern fish | *Benia* |
| Shiny loosejaw | *Aristostomias* |
| Hatchet fish | *Agyropelecus* |
| and other fish | *Pachystomias* |
| | *Malacosteus* |
| Midwater fish | Cyclothone |
| | *Neoscopelus* |
| | *Tarletonbeania* |

Said *luciferase* of the invention is selected in the group consisting of bacteria, mushrooms, Dinoflagellates, Cnidaria (coelenterates), anelids, crustacea, insects, Echinoderms, Chordates, fishes.

In a preferred embodiment, said *luciferase* is isolated from Cnidaria and is selected in the group consisting of jellyfish, hydroid, sea pansy, ctenophores. All the following described luciferases isolated from Cnidaria, and their variants and mutants thereof, are encompassed in the scope of the invention. Representative of Cnidaria is *Renilla* also known as sea pansies, which are members of the class of coelenterates *Anthozoa*, which includes other bioluminescent genera, such as *Cavarnularia, Ptilosarcus, Stylatula, Acanthoptilum*, and *Parazoanthus.* Bioluminescent members of the *Anthozoa* genera contain luciferases and luciferins that are similar in structure (see, e.g., Cormier *et al.*, 1973 ; see, also Ward *et al.*, 1975). The luciferases and luciferins from each of these anthozoans crossreact and produce a characteristic blue luminescence. *Renilla luciferase* and the other coelenterates and ctenophore luciferases, such as the æquorin photoprotein, use imidazopyrazine substrates, particularly the substrates generically called coelenterazine. *Renilla luciferase* does not, however, have bound oxygen, and thus requires dissolved oxygen in order to produce light in the presence of a suitable luciferin substrate. Since *Renilla luciferase* acts as a true enzyme (i.e., it does not have to be reconstituted for further use) the resulting luminescence can be longlasting in the presence of saturating levels of luciferin. Also, *Renilla luciferase* is relatively stable to heat. *Renilla luciferase*, DNA encoding *Renilla luciferase*, and use of the DNA to produce recombinant *luciferase*, as well as DNA encoding *luciferase* from other coelenterates, are well known and available (see, e.g., US patents Nos. 5,418,155 and 5,292,658 ; see, also, Prasher *et al.*, 1985 ; Cormier, 1981 ; Charbonneau *et al.*, 1979 ; Ward *et al.*, 1979 ; Lorenz *et al.*, 1981 ; Hori *et al.*, 1977 ; Inouye *et al*., 1975 ; Matthews *et al*., 1979). According to a preferred embodiment, said *luciferase* is isolated from sea pansy and is *Renilla reniformis luciferase* that uses the imidazolopyrazine substrate (coelenterazine) to emit blue light (460-510 nm). According to another preferred embodiment, said *luciferase* is isolated from jellyfish and is *Aequorea luciferase.* According to another preferred embodiment, said *luciferase* is isolated from hydroid and is *Obelia luciferase.*

In another preferred embodiment, said *luciferase* is isolated from insects and is selected in the group consisting of Coleopterids (beetles), firefly, click beetles, railroad worm, diptera (flies) . All of the following described luciferases isolated from insects, and their mutants and variants thereof are encompassed in the scope of the invention. The biochemistry of firefly bioluminescence was the first bioluminescence generating system from insects to be characterized (see, e.g., Wienhausen *et al.*, 1985) and it is commercially available (e.g., from PROMEGA Corporation, Madison, Wis., see, e.g., Leach *et al.*, 1986). These species include members of the genera *Photinus*, Photurins and *Luciola.* Further, the bioluminescent reaction produces more light at 30°C than at 20°C, the *luciferase* is stabilized by small quantities of bovine serum albumin, and the reaction can be buffered by tricine. DNA clones encoding luciferases from various insects and their use to produce the encoded *luciferase* are well known. For example, DNA clones that encode *luciferase* from *Photinus pyralis, Luciola cruciata* (see, e.g., de Wet *et al.*, 1985 ; de Wet *et al.*, 1986 ; US patent No. 4,968,613) are available. In addition to the wild type *luciferase,* modified insect luciferases have been prepared. For example, heat stable *luciferase* mutants, DNA-encoding the mutants, vectors and transformed cells for producing the luciferases are available. A protein with 60% amino acid sequence homology with luciferases *from Photinus pyralis, Luciola mingrelica, L. cruciata or L. lateralis* and having *luciferase* activity is available (see, e.g., International PCT application No. WO95/25798). It is more stable above 30°C than naturally-occurring insect luciferases and may also be produced at 37°C or above, with higher yield. Modified luciferases that generate light at different wavelengths (compared with native *luciferase*) are known, and thus may be selected for their color-producing characteristics. For example, synthetic mutant beetle *luciferase*(s) and DNA encoding such luciferases that produce bioluminescence at a wavelength different from wild-type *luciferase* are known (PROMEGA Corp., International, PCT application No. W095/18853). The mutant beetle *luciferase* has an amino acid sequence differing from that of the corresponding wild-type *Luciola cruciata* (see, e.g., US patents Nos. 5,182,202, 5,219,737, 5,352,598) by a substitution(s) at one or two positions. The mutant *luciferase* produces bioluminescence with a wavelength of peak intensity that differs by at least 1 nm from that produced by wild-type luciferases. Other mutant luciferases have also been produced (see US 5,219,737 and US 5,330,906). These luciferases produce light with colors that differ from wild-type. These mutant luciferases catalyze *luciferin* to produce red (λ 609 nm and 612 nm), orange (λ595 and 607 nm) or green (λ558 nm) light. The other physical and chemical properties of mutant *luciferase* are substantially identical to native wild-type-luciferase. Thermostable luciferases are also available (see US 5,229,285 ; see also International PCT application No. WO 95 27598). These mutant luciferases as well as the wild type luciferases are among those preferred herein, particularly in the case where a variety of colors are desired or when stability at higher temperatures is desired. The firefly luciferin is a benzothiazole. Analogs of this luciferin and synthetic firefly luciferins are also known to those of skill in the art (see, e.g., US patents Nos. 5,374,534 and 5,098,828). The reaction catalyzed by firefly luciferases and related insect luciferases requires ATP, Mg²⁺ as well as molecular oxygen. Luciferin must be added exogenously. Firefly *luciferase* catalyzes the firefly luciferin activation and the subsequent steps leading to the excited product. The luciferin reacts with ATP to form a luciferyl adenylate intermediate. This intermediate then reacts with oxygen to form a cyclic luciferyl peroxy species, similar to that of the coelenterate intermediate cyclic peroxide, which breaks down to yield CO₂ and an excited state of the carbonyl product. The excited molecule then emits a yellow light ; the color, however, is a function of pH. As the pH is lowered the color of the bioluminescence changes from yellow-green to red. Different species of fireflies emit different colors of bioluminescence so that the color of the reaction will be dependent upon the species from which the *luciferase* is obtained. Addition of ATP and luciferin to a reaction that is exhausted produces additional light emission. Thus, the system, once established, is relatively easily maintained. Therefore, this system is highly suitable for use herein in embodiments in which a sustained glow is desired. In a preferred embodiment said *luciferase* isolated from firefly is selected from *Photinus luciferase* and from *Photuris luciferase.* In a preferred embodiment, said *luciferase* of the invention is *Photinus luciferase* that uses the benzothiazoyl-thiazole as the substrate (luciferin) and emits light at 550-580 nm. In another preferred embodiment, said *luciferase* is isolated from Crustacea and is *vargula luciferase.*

The preferred luciferases are those that are used to generate the fusion protein of the invention or the above described luciferases and/or *luciferase* that have minor sequence variations. Such minor sequence variations include, but are not limited to, minor allelic or species variations and insertions or deletions of residues, particularly cysteine residues. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (see, e.g., Watson *et al.*, 1987). Thus, for purposes herein the biological activity of a *luciferase* is its oxygenase activity whereby, upon oxidation of a substrate, light is produced. Other substitutions are also permissible and may be determined empirically or in accord with known conservative substitutions. Any such modification of the polypeptide may be effected by any means known to those of skill in this art. Naturally-occurring luciferases of the invention (including photoproteins), and *luciferase* variants produced by recombinant DNA, and mutated or modified variants thereof, retain the ability to generate light in the presence of an appropriate substrate, co-factors and activators or any other such protein that acts as a catalyst to oxidize a substrate, whereby light is produced.

As used herein, bioluminescence substrate refers to the compound that is oxidized in the presence of a *luciferase,* and any necessary activators, and generates light. These substrates are referred to as luciferins, which are substrates that undergo oxidation in a bioluminescence reaction. These bioluminescence substrates include any luciferin or analog thereof or any synthetic compound with which a *luciferase* interacts to generate light. Preferred substrates are those that are oxidized in the presence of a *luciferase* or protein in a light-generating reaction. Thus, bioluminescence substrates include those compounds that those of skill in the art recognize as luciferins. Such molecules include the naturally-occurring substrates, modified forms thereof, and synthetic substrates (see, e.g., US patents Nos. 5,374,534 and 5,098,828). Exemplary luciferins include those described herein, as well as derivatives thereof, analogs thereof, synthetic substrates, such as dioxetanes (see, e.g., US patents Nos. 5,004,565 and 5,455,357), and other compounds that are oxidized by a *luciferase* in a light-producing reaction (see, e.g., US patents Nos. 5,374,534, 5,098,828 and 4,950,588). Such substrates also may be identified empirically by selecting compounds that are oxidized in bioluminescent reactions. Luciferins, for example, include firefly luciferin, *Cypridina* (also known as *Vargula*) luciferin (*coelenterazine*), bacterial luciferin, as well as synthetic analogs of these substrates or other compounds that are oxidized in the presence of a *luciferase* in a reaction that produces bioluminescence.

The bioluminescence generating systems also require additional components discussed herein and known to those of skill in the art. All bioluminescent reactions require molecular oxygen in the form of dissolved or bound oxygen. Thus, molecular oxygen, dissolved in water or in air or bound to a photoprotein, is the activator for bioluminescence reactions. Depending upon the form of the components, other activators include, but are not limited to, ATP (for firefly *luciferase*), flavin reductase (bacterial systems) for regenerating FMNH₂ from FMN, and Ca²⁺ or other suitable metal ion (*æquorin*).

Most of the systems provided herein will generate light when the *luciferase* and luciferin are mixed and exposed to air or water. The systems that use photoproteins that have bound oxygen, such as *æquorin*, however, will require exposure to Ca²⁺ (or other suitable metal ion), which can be provided in the form of an aqueous composition of a calcium salt. In these instances, addition of Ca²⁺ (or other suitable metal ion) to a mixture of *luciferase* (*æquorin*) and luciferin (such as coelenterazine) will result in generation of light. The *Renilla* system and other *Anthozoa* systems also require Ca²⁺ (or other suitable metal ion).

The active recombinant fusion protein of the invention comprises a *luciferase*, or a fragment, or a variant thereof fused to a selection marker protein, or a fragment or a variant thereof. By "selection marker protein fragment", it is meant any biologically active fragment of a selection marker protein, that is to say any fragment of a natural or variant protein having a selection marker activity. As used herein, selection marker means a protein or a peptide that allows one to select for or against a molecule or a cell that contains it, often under particular conditions i.e. in presence of a selective agent. These selection marker proteins include but are not limited to products which provide resistance against otherwise toxic compounds (e.g., antibiotics). For example, the ampicillin or the neomycin resistance genes constitute genes encoding for selection markers of the invention. Those selection markers can be either positive or negative (see Capecchi et al. (1989), US 5 631 153).

According to a preferred embodiment, said selection marker protein is a positive selection marker protein encoded by a gene selected in the group consisting of antibiotic resistance genes, hisD gene, *Hypoxanthine phosphoribosyl transferase* (HPRT) gene, guanine-phosphoribosyl-transferase (Gpt) gene. Said antibiotic resistance gene is selected in the group consisting of hygromycin resistance genes, neomycin resistance genes, tetracyclin resistance genes, ampicillin resistance genes, kanamycin resistance genes, phleomycin resistance genes, bleomycin resistance genes, geneticin resistance genes, carbenicillin resistance genes, chloramphenicol resistance genes, puromycin resistance genes, blasticidin-S-deaminase genes. In a preferred embodiment, said antibiotic resistance gene is a hygromycin resistance gene, preferably an *Escherichia coli* hygromycin-B phosphotransferase (hpt) gene. In this case the selective agent is hygromycin. In another preferred embodiment, said antibiotic resistance gene is a neomycin resistance gene. Said neomycin resistance gene is chosen in respect to the cellular host in which the fusion protein is expressed. For an expression restricted to procaryotic cells, the neomycin resistance gene encoded by the Tn10 transposon is preferred; in this case, the selective agent is kanamycin. More preferably the Tn5 neomycin resistance gene is used; such gene allowing one to perform the selection both in procaryotic and eukaryotic cells. In this latter case, the selective agent is G418. In another embodiment, the positive selection marker protein of the invention is His D; in this case, the selective agent is Histidinol. In another embodiment, the positive selection marker protein of the invention is Hypoxanthine phosphoribosyl transferase (HPRT) or Hypoxanthine guanosyl phosphoribosyl transferase (HGPRT); in this case, the selective agent is Hypoxanthin. In another embodiment, the positive selection marker protein of the invention is guanine-phosphoribosyl-transferase (Gpt); in this case, the selective agent is xanthin.

It is also in the scope of the invention to use negative selection marker proteins. For example, the genes encoding for such proteins are the HSV-TK gene ; in this case the selective agent is Acyclovir-Gancyclovir. For example, the genes encoding for such proteins are the Hypoxanthine phosphoribosyl transferase (HPRT) gene or the guanine-phosphoribosyl-transferase (Gpt) gene; in these cases the selective agent is the 6-Thioguanine. For example, the genes encoding for such proteins include the cytosine deaminase gene; in this case the selective agent is the 5-fluoro-cytosine.

In a first preferred embodiment, the fusion protein of the invention comprises *Photinus luciferase* (Luc) or a fragment or variant thereof, fused to an *Escherichia coli* Hygromycin-B phosphotransferase (hpt) gene or a fragment or variant thereof. The sequence of this fusion protein named *Luht* is SEQ ID N° 2.

In a second preferred embodiment, the fusion protein of the invention comprises *Renilla reniformis luciferase* (Ruc) or fragment or variant thereof, fused to an *Escherichia coli* hygromycin B phosphotransferase (hpt) gene or a fragment or variant thereof. The sequence of this fusion protein named *Ruht* is SEQ ID No. 4.

In a third preferred embodiment, the fusion protein of the invention comprises *Photinus luciferase* (Luc) or a fragment or variant thereof, fused to a Tn5 neomycin resistance gene or a fragment or variant thereof. The sequence of this fusion protein named *LeoK* is SEQ ID No. 6.

In a fourth preferred embodiment, the fusion protein of the invention comprises *Renilla reniformis luciferase* (Ruc) or a fragment or variant thereof, fused to a Tn5 neomycin resistance gene or a fragment or variant thereof. The sequence of this fusion protein named ReoK is SEQ ID No. 8.

It is also in the scope of the invention to furnish fusion proteins that have linker peptides between the *luciferase* and the selection marker protein. In a preferred embodiment, this linker peptide is long enough to permit the *luciferase* and the selection marker protein to be functionaly active, or to code for an additional biological function, or to possess an activity. For example, the linker peptide between the luc and neo genes in Leok and Reok has a prokaryotic promoter activity.

The present invention also relates to the recombinant polynucleotide encoding for a protein fusion of the invention. In a first embodiment the recombinant polynucleotide of the invention is the polynucleotide of sequence SEQ ID No. 1 encoding for the fusion protein *Luht* of sequence SEQ ID No. 2. In a second embodiment the recombinant polynucleotide of the invention is the polynucleotide of sequence SEQ ID No. 3 encoding for the fusion protein *Ruht* of sequence SEQ ID No. 4. In a third embodiment the recombinant polynucleotide of the invention is the polynucleotide of sequence SEQ ID No. 5 encoding for the fusion protein *Leok* of sequence SEQ ID No. 6. In a fourth embodiment the recombinant polynucleotide of the invention is the polynucleotide of sequence SEQ ID No. 7 encoding for the fusion protein *Reok* of sequence SEQ ID No. 8.

The present invention also relates to the recombinant polynucleotide comprising a polynucleotide selected among (a) the polynucleotides of sequences SEQ ID No. 1, SEQ ID No. 3, SEQ ID No. 5, SEQ ID No. 7 ; (b) the polynucleotides presenting at least 80% identity after optimal alignment with the polynucleotide of step (a) ; and (c) complementary sequence or RNA sequence corresponding to a polynucleotide of step (a) or (b).

As used herein, polynucleotides include reference to a desoxyribonucleotide or ribonucleotide polymer in either single or double stranded form. The polynucleotides of the invention (i.e. nucleic acid sequences of the invention) are recombinant polynucleotides that have been generated either recombinantly or synthetically.

As used herein, "percentage of identity" between two nucleic acids sequences or two amino acids sequences, means the percentage of identical nucleotides, or amino acids respectively, between the two sequences to be compared, obtained with the best alignment of said sequences, this percentage being purely statistical and the differences between these two sequences being randomly spread over the nucleic acid or amino acid sequences. As used herein, "best alignment" or "optimal alignment", means the alignment for which the determined percentage of identity (see below) is the highest. Sequence comparison between two nucleic acids or amino acids sequences are usually realised by comparing sequences that have been previously aligned according to the best alignment; this comparison is realised on segments or windows of comparison in order to identify and compared the local regions of similarity. The best sequence alignment to perform this comparison can be realised, in addition to a manual approach, by using the local homology algorithm developed by Smith and Waterman (1981), by using the local homology algorithm developed by Neddleman and Wunsch (1970), by using the method of similarities developed by Pearson and Lipman (1988), by using computer softwares using appropriate algorithms (for example GAP, BESTFIT, BLAST P, BLAST N, FASTA, TFASTA in the Wisconsin Genetics software Package, Genetics Computer Group, 575 Science Dr., Madison, WI USA). To get the best alignment, one can preferably use BLAST software, with the BLOSUM 62 matrix, or the PAM or PAM 250 matrix. The percentage identity between two sequences of nucleic acids or amino acids is determined by comparing these two sequences optimally aligned, the nucleic acid or the amino acid sequences being able to comprise additions or deletions in respect to the reference sequence in order to obtain the optimal alignment between these two sequences. The percentage of identity is calculated by determining the number of identical positions between these two sequences, and dividing this number by the total number of compared positions, and by multiplying the result obtained by 100 to get the percentage of identity between these two sequences.

As used herein nucleic acids sequences having a percentage of identity of at least 80%, preferably, at least 82%, 85%, 87%, 90%, 92%, 95%, 97%, 98%, 99%, 99.5% after optimal alignment, means nucleic acid sequences having with regard to the reference sequence, modifications such as deletions, truncations, insertions, chimeric fusions, and/or substitutions, most especially point mutations, the nucleic sequence of which presents at least 80%, preferably, at least 82%, 85%, 87%, 90%, 92%, 95%, 97%, 98%, 99%, 99.5% identity after optimal alignment with the nucleic acid sequence of reference.

The invention also furnishes a DNA cassette comprising a promoter operably linked to a polynucleotide of the invention. A DNA cassette is a nucleic acid construct generated recombinantly or synthetically, with a series of specified nucleic acid elements which permit transcription of a particular nucleic acid in a target cell. The DNA cassette can be part of a vector or a nucleic acid fragment.

A "promoter" or a "promoter sequence" is a DNA regulatory region capable of binding RNA polymerase in a cell and initiating transcription downstream (3'direction) of a coding sequence. Within the promoter sequence will be found a transcription initiation site, as well as protein binding domains responsible for the binding of RNA polymerase. Eukaryotic promoters will often, but not always, contain TATA boxes and CAT boxes. Various promoters, including ubiquitous or tissue-specific promoters, and inducible and constitutive promoters may be used to drive the expression of the protein fusion gene of the invention. "operatively linked" as used herein, includes reference to a functional linkage between a promoter and a second sequence (i.e. a polynucleotide of the invention), wherein the promoter sequence initiates and mediates the transcription of said DNA sequence cooresponding to the second sequence. Generally operatively linked means that the nucleic acid sequences being linked are contiguous and, where necessary to join two protein coding regions, contiguous and in the same reading frame.

It is also a goal of the invention to furnish a vector comprising a polynucleotide of the invention (i.e. a cloning vector) or an expression vector comprising at least one DNA cassette of the invention. A "vector" is a replicon in which another polynucleotide segment is attached, so as to bring the replication and/or expression to the attached segment. Examples of vectors include plasmids, phages, cosmids, phagemid, yeast artificial chromosomes (YACs), bacterial artificial chromosomes (BACs), human artificial chromosomes (HACs), viral vectors, such as adenoviral vectors, retroviral vectors, and other DNA sequences which are able to replicate or to be replicated *in vitro* or in a host cell, or to convey a desired DNA segment to a desired location within a host cell. A vector can have one or more restriction endonuclease recognition sites at which the DNA sequences can be cut in a determinable fashion without loss of an essential biological function of the vector, and into which a DNA fragment can be spliced in order to bring about its replication and cloning. Vectors can further provide primer sites (e.g. for polymerase chain reaction, PCR), transcriptional and/or translational initiation and/or regulation sites, splicing sites, recombinational signals, replicons, selectable markers, etc. Beside the use of homologous recombination or restriction enzymes to insert a desired DNA fragment into the vector, UDG cloning of PCR fragments (US Pat. No. 5,334,575), T:A cloning, and the like can also be applied. The cloning vector can further contain a selectable marker suitable for use in the identification of cells transformed with the cloning vector.

The disclosed vectors can be used to transiently transfect or transform host cells, or can be integrated into a host cell chromosome. Preferably, however, the vectors can include sequences that allow replication of the vector and stable or semi-stable maintenance of the vector in the host cell. Many such sequences for use in various cells (that is, eukaryotic and prokaryotic cells) are known and their use in vectors routine. Generally, it is preferred that replication sequences known to function in host cells of interest be used.

The fusion gene present in a DNA cassette or in an expression vector can be expressed using any suitable expression sequences. Numerous expression sequences are known and can be used for expression of the fusion genes. Expression sequences can generally be classified as promoters, terminators, and, for use in eukaryotic cells, enhancers. Expression in prokaryotic cells also requires a « *Shine-Dalgarno* » sequence just upstream of the coding region for proper translation initiation. Promoters suitable for use with prokaryotic hosts illustratively include the β-lactamase and lactose promoter systems, tetracycline (tet) promoter, alkaline phosphatase promoter, the tryptophan (trp) promoter system and hybrid promoters such as the tac promoter. However, other functional bacterial promoters are suitable. Their nucleotide sequences are generally known. Suitable promoting sequences for use with yeast hosts include, for example, the promoters for 3-phosphoglycerate kinase, enolase, glyceraldehyde-3-phosphate dehydrogenase, hexokinase. Examples of inducible yeast promoters suitable for use in the vectors of the invention include, for example, the promoter regions for alcohol dehydrogenase 2, isocytochrome C, acid phosphatase. Yeast enhancers also are advantageously used with yeast promoters. Preferred promoters for use in mammalian host cells include promoters from polymoma virus, Simian Virus 40 (SV40), adenovirus, retroviruses, *hepatitis* B virus, herpes *simplex* virus (HSV), Rous *sarcoma* virus (RSV), mouse mammary tumor virus (MMTV), and most preferably cytomegalovirus (CMV), or from heterologous mammalian promoters such as the β-actin promoter. Transcription of the reporter gene by higher eukaryotes can be increased by inserting an enhancer sequence into the vector. Many enhancer sequences are now known from mammalian genes (globin, elastase, albumin, and insulin) or from eukaryotic cell viruses (SV40, CMV). The disclosed vectors preferably also contain sequences necessary for accurate 3'end termination ; in eukaryotic cells, this would be a polyadenylation signal. In prokaryotic cells, this would be a transcription terminator.

The disclosed vectors, the components of which are described above, can be constructed using well established recombinant DNA techniques (see, for example, Sambrook *et al.*, 1989).

In a preferred embodiment, the invention relates to a gene targeting vector comprising at least the following nucleotide sequences in the following order:(i) a first sequence homologous to the targeted gene;(ii)optionally a genetic element;(iii)an internal ribosome entry site (IRES);(iv)a polynucleotide or a DNA cassette of the invention; (v) a polyadenylation site;(vi)a second sequence homologous to the targeted gene.

The invention also relates to a gene trap vector comprising at least the following nucleotide sequences in the following order (i)a genetic element; (ii) an internal ribosome entry site (IRES); (iii) a polynucleotide of the invention; (vi) optionally, a polyadenylation site.

The invention also relates to a gene trap vector comprising at least the following nucleotide sequences in the following order (i)a promoter ; (ii) an internal ribosome entry site (IRES); (iii) a polynucleotide of the invention; (vi) optionally, a polyadenylation site.

The invention also relates to a gene trap vector comprising at least the following nucleotide sequences in the following order (i)an acceptor splicing site; (ii) an internal ribosome-entry site (IRES); (iii) a polynucleotide of the invention; (iv) a donor splicing site.

In particular embodiments, the fusion genes of the first invention are operatively joined to an internal ribosome-entry site (IRES). Different IRES may be used, including the encephalomyocarditis virus IRES (Mountford and Smith, 1995), the poliovirus IRES (Pelletier and Sonenberg, 1988), the aphtovirus IRES (Lopez de Quinto and Martinez-Salas, 1999), the hepatitis C virus IRES (Collier *et al.*, 1998), or the IRES from the VEGF gene (Huez *et al.*, 1998).

As used herein, "Genetic element" means any genomic DNA, recombinant DNA, or cDNA sequences, the presence of which in the vector will allow the vector to be correctly targeted or to be functional. For example, in the case of Knock-Out targeting vector, such "genetic element" can encode for a mutated exon(s) comprising a point mutation, a deletion, insertion, substitution. For example, such "genetic element" can encode for a protein of interest having for example a therapeutic, a diagnostic or a research interest. Such genetic element can comprise any of the genetic elements necessary for the correct expression of this protein of interest in the host cell. Examples of such proteins of interest are the autofluorescence protein such as GFP (green fluorescent protein), the site-specific recombinases (Cre, FLP,...), or peptidic tags.

Host cells can be transformed with the disclosed polynucleotides, DNA cassette, or vectors of the invention using any suitable means and cultured in conventional nutrient media modified as is appropriate for inducing promoters, selecting transformants and/or detecting expression. Suitable culture conditions for host cells, such as temperature and pH, are well known. In a preferred embodiment, transformed cells of the invention are cultured in presence of a selective agent as previously described in the cell culture media. Such polynucleotides, DNA cassettes, or vectors of the invention can be introduced into a host cell either in *vivo* or *in vitro* using known techniques, such as CaPO₄ precipitation, electroporation, cationic lipofection, use of artificial viral envelopes, direct injection (e.g., intravenous, intraperitoneal or intramuscular), and micro-injection into a zygote or a pronucleus of a zygote. Thus, the invention relates to an isolated transgenic host cell transformed by a polynucleotides, DNA cassette, or vectors of the invention.

A "host", as the term is used herein, includes prokaryotic or eukaryotic organisms that can be genetically engineered. For examples of such hosts, see Sambrook *et al.*, (1989). The cell of the invention is characterized by the fact that the protein fusion encoded by said polynucleotide, said DNA cassette, or said vector, is expressed and biologically active in said cell.

In a preferred embodiment, the cell of the invention is selected among eukaryotic and prokaryotic cells. Preferably, the cell of the invention is an eukaryotic cell selected among human cells, murine cells, yeast cells. More preferably it is a human cell (such as HeLa cells). In another embodiment, the cell of the invention is a prokaryotic cell selected among bacteria such as for example *Escherichia coli* and *Bacillus subtilis.*

In a preferred embodiment, the targeting vector of the invention, or a fragment thereof, is integrated by homologous recombination in at least one targeted locus of the genome of a host cell of the invention. To perform such homologous recombination, it is preferable that sequences of homology are present at both extremities of the linearized vector of the invention, or the polynucleotide or the DNA cassette of the invention.

In another embodiment, said gene trap vector of the invention, or a fragment thereof, is integrated in site(s) of the genome of the host cell of the invention chosen among polyA sites, gene exons and gene promoters. The specific embodiment allows to perform gene trapping which is a general method for mutagenesis based on random integration of a DNA fragment encoding for a reporter gene or a selectable marker gene (Hill and Wurst, 1993). Three gene trap methods are commonly used, the polyA trap based method, the promoter trap based method, the exon trap based method.

In another embodiment the polynucleotide, the DNA cassette or the vector of the invention is randomly integrated in at least one locus of the genome of said isolated transgenic host cell. In another embodiment the polynucleotide, the DNA cassette or the vector of the invention is maintained in an episomal form in said isolated transgenic host cell.

The present invention also relates to a transgenic organism, comprising at least one cell according to the invention. An « organism » as the term is used herein, includes but is not limited to, bacteria, yeast, animal, plants. Among the animals, one can designate mammals, such as rodents, primates, except humans, farm animals. In a preferred embodiment, the animal is a mouse, a rat, a Guinea pig, a hamster, a rabbit, a pig, a cow, a horse, a goat, a sheep. In a preferred embodiment the animal is a mouse. In another preferred embodiment the organism is a yeast.

The invention also relates to an *in vitro* method of selecting cells into the genome of which a genetic element has been targeted into at least one predefined locus, said method comprising the steps of (a) introducing a vector of the invention or a fragment into a host cell ; (b) culturing said cell in the presence of an appropriate selective agent under conditions whereby said vector or fragment thereof integrates by homologous recombination into at least one predefined locus of the genome of said cell, and said cell expresses the fusion protein encoded by said vector or a fragment thereof ; (c) contacting cells with appropriate luciferin ; (d) identifying cells expressing the *luciferase* activity of said fusion protein by photon emission detection, then selecting cells.

The invention also relates to an *in vitro* method for trapping an unknown gene, said method comprising the steps of (a) introducing a vector of the invention or a fragment thereof into a host cell ; (b) culturing said cell in the presence of an appropriate selective agent under conditions whereby said vector or fragment thereof integrates into the genome of said cells, said cells express the fusion protein encoded by said vector or a fragment thereof ; (c) contacting cells with luciferin ; (d) identifying cells expressing *luciferase* activity of said fusion protein by photon emission detection, then selecting cells ; and(e) isolation and/or identification of the gene(s) in which the trapping vector integrates. In a preferred embodiment, the *luciferase* activity is further quantified to estimate the level of expression of said unknown gene in said cell.

It is also a goal of the invention to furnish kits for transgenic cell selection and/or for monitoring gene expression, said kits comprising: (a) compound selected in the group consisting of polynucleotide, vector and DNA cassette of the invention ; (b) at least one luciferin, which is a substrate for the *luciferase* activity of the fusion protein encoded by said compound ; (c) at least one appropriate selective agent.

Finally, the invention relates to the use of a fusion protein and/or a polynucleotide and/or a vector and/or a DNA cassette and/or a kit of the invention for transgenic cell selection and/or monitoring gene expression. More preferably, the fusion protein and/or a polynucleotide and/or a vector and/or a DNA cassette and/or a kit of the invention is dedicated to perform gene targeting, gene tagging, gene knock-out (KO), gene knock-in (KI), polyA trap, exon trap, promoter trap experiments.

The fusion genes and methods of the invention offer a novel opportunity for easy and fast identification of targeted clones. The use of *luciferase* genes as the reporter component allows identification of candidate clones directly on master dishes in living conditions, therefore reducing the cell manipulations and cell culture time length. This also permits the application of the invention to cells where homologous recombination frequency is low, preferably somatic cells, and more preferably human cells in order to perform identification and/or functional analysis of expressed genes.

Unless defined otherwise, all technical and scientific terms used herein have the same meaning as is commonly understood by one of skill in the art to which this invention belongs.

The figures and examples presented below are provided as a further guide to the practitioner of ordinary skill in the art and are not to be construed as limiting the invention in anyway.

### FIGURES

**Figure 1 : Comparison of β-Geo- and *luciferase*selectable marker fusion gene-based vectors in gene targeting experiments.**
   **(A). β-Geo-based approach.** Assays for β-galactosidase activity are performed by X-GAL histochemistry after fixation and permeabilization of cells, and therefore require random picking and duplication of colonies.
   **(B). *Luciferase*-selectable marker fusion gene-based approach.** The *luciferase* activity is monitored on master dishes in living conditions by low light video imaging and only positive recombinant lines are picked.

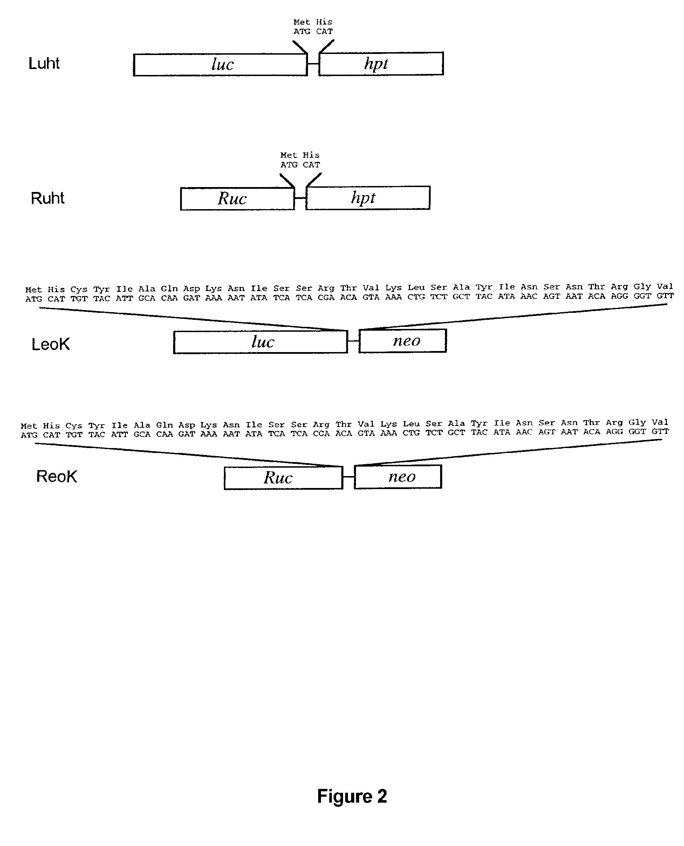

**Figure 2 : Schematic diagram showing the fusion genes according to the invention.** The linker sequence between the *luc* and *neo* genes in *LeoK* and between the *Ruc* and *neo* genes in *ReoK* has a prokaryotic promoter activity. In consequence, *LeoK* and *ReoK* containing plasmids confer kanamycin resistance to *Escherichia coli.*

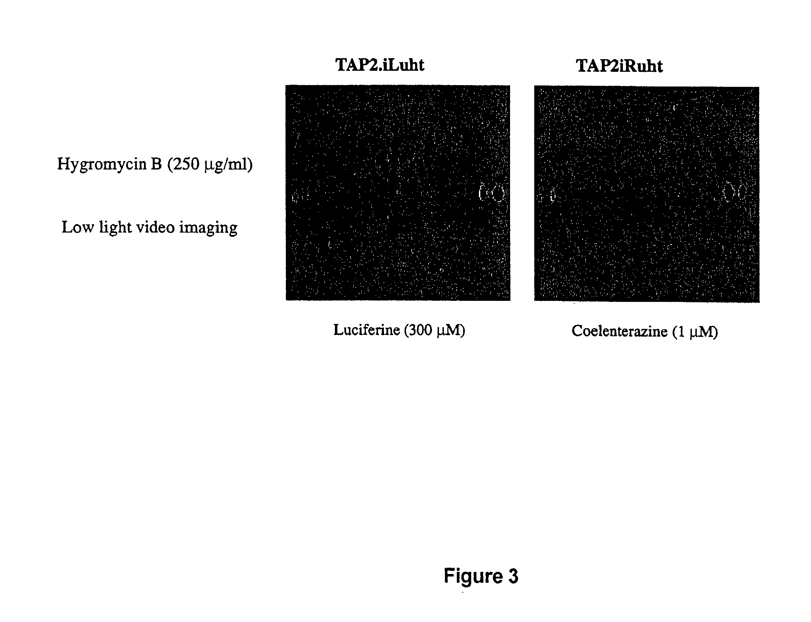

**Figure 3 : Luht and Ruht genes confer both *luciferase* activity and hygromycin B resistance.** Constructs expressing Luht (left) and Ruht (right) were electroporated into murine F9 cells. After 10 days of culture in presence of hygromycin B (250 µg/ml), *luciferase* expressing clones were identified by low light video imaging in presence of the corresponding substrates luciferin or coelenterazine, respectively.

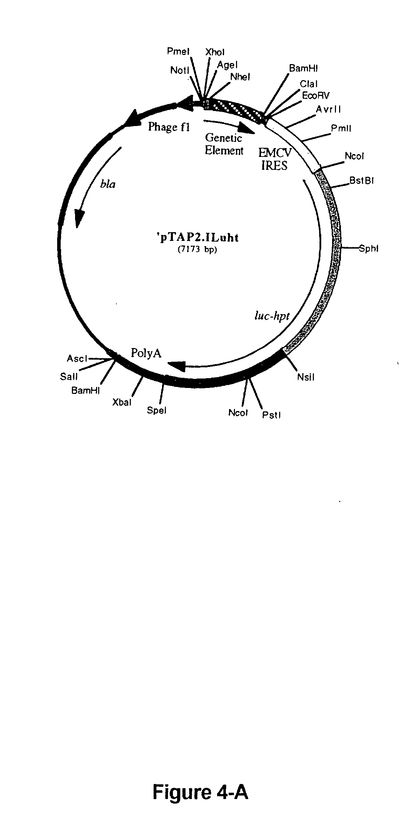

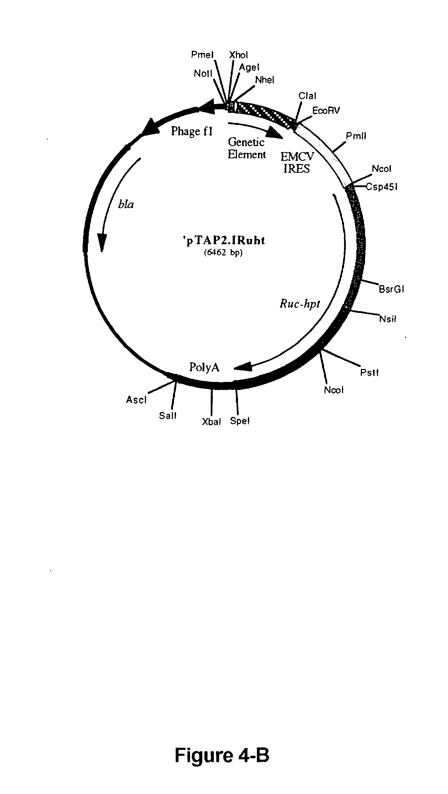

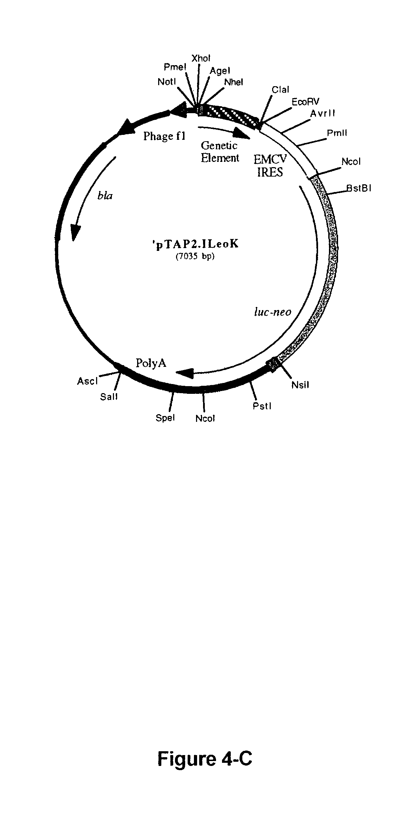

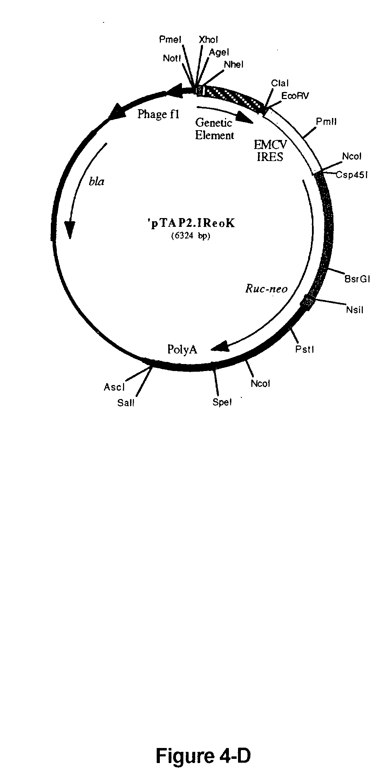

**Figure 4 : maps of the plasmids according to the invention.**

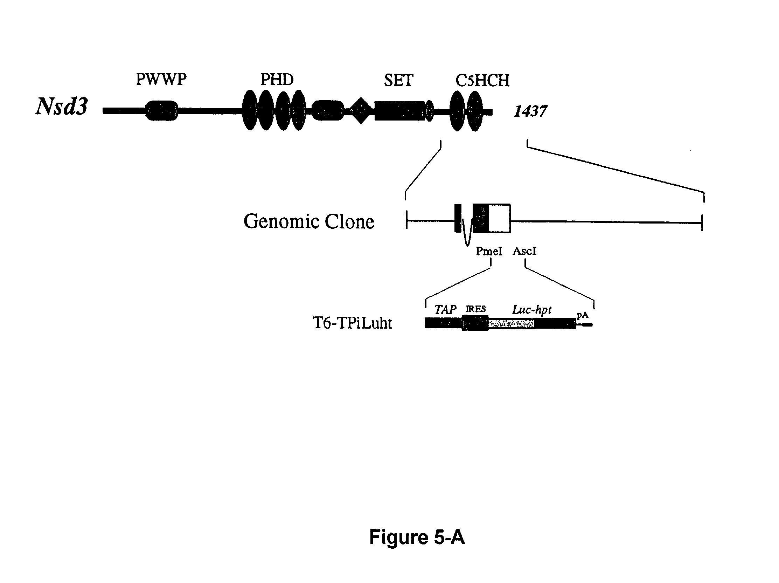

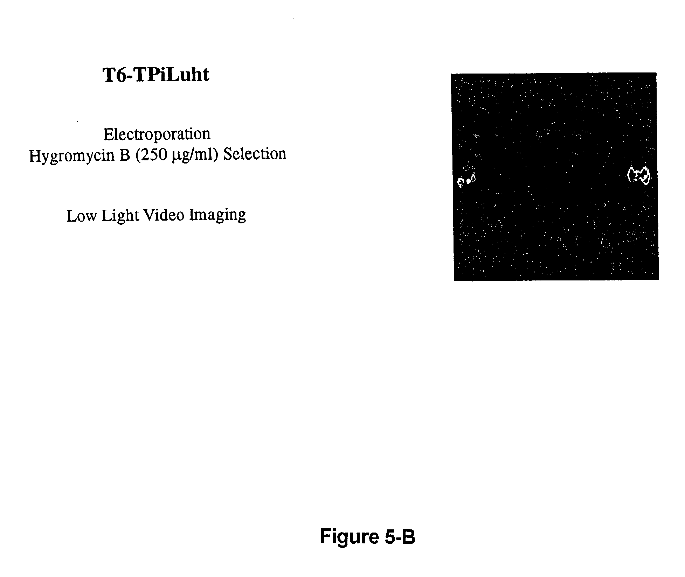

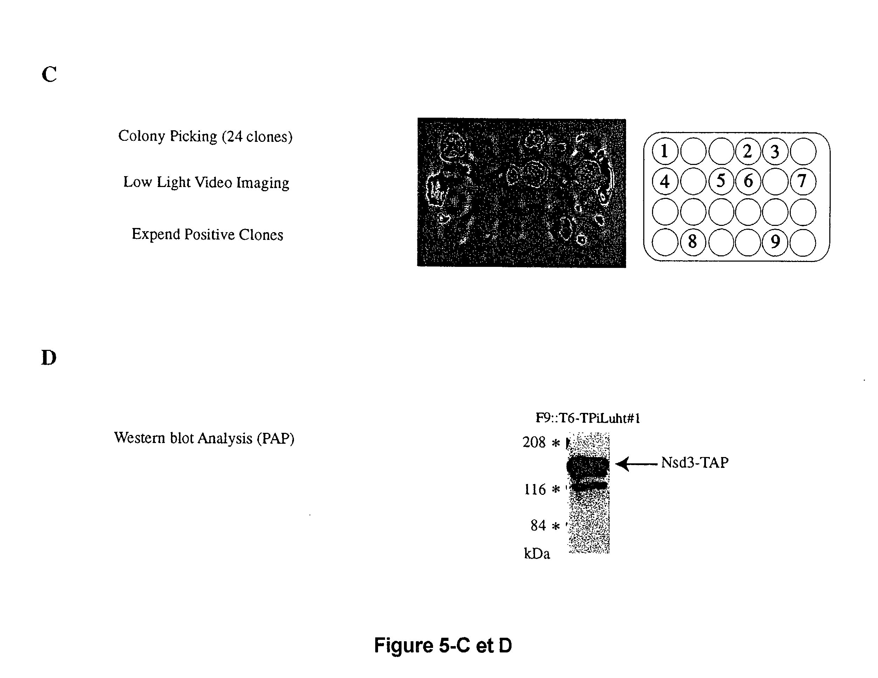

**Figure 5 : Experimental procedure in which a tag (the TAP tag - Rigaut *et al.*, 1999) is targeted at the 3'-end of the *Nsd3* coding sequence in F9 cells.**
   **(A).** Schematic representation of the Nsd3 protein and its different domains (Angrand *et al.* (2001) Genomics, 74:in press). The genomic clone used contains the last two exons (exons 22 and 23) of the *Nsd3* gene, encoding the fifth PHD finger and the C5HCH motif. ET recombination to remove the Nsd3 STOP codon and to introduce two restriction sites (PmeI and AscI), was obtained by chloramphenicol selection in the *sbcA E. coli* strain (Angrand *et al.*, 1999). In a second in *vitro* restriction/ligation step, a cassette containing a modified TAP tag (Rigaut *et al.*, 1999) and a promoterless luc-hpt gene was inserted to generate the final targeting construct (as described in Angrand *et al.*, 1999).
   **(B).** The targeting construct was introduced by electroporation into F9 cells, and after hygromycin B (250 µg/ml)-selection, candidates were identified by low light video imaging in presence of luciferin.
   **(C).** 24 luciferases expressing clones were picked and transfered into a 24-well dish. After growth, the clones were re-tested for *luciferase* activity and 9 clones were further analyzed.
   **(D).** Western blot analysis of one clone (F9::T6-TpiLuhtN°1) using a peroxidase anti-peroxidase antibody (PAP) recognizing the IgG-binding domain of the *S. aureus* protein A included in the TAP tag. The clone F9::T6-TpiLuhtN°1 expresses a Nsd3-TAP fusion protein of the expected size.

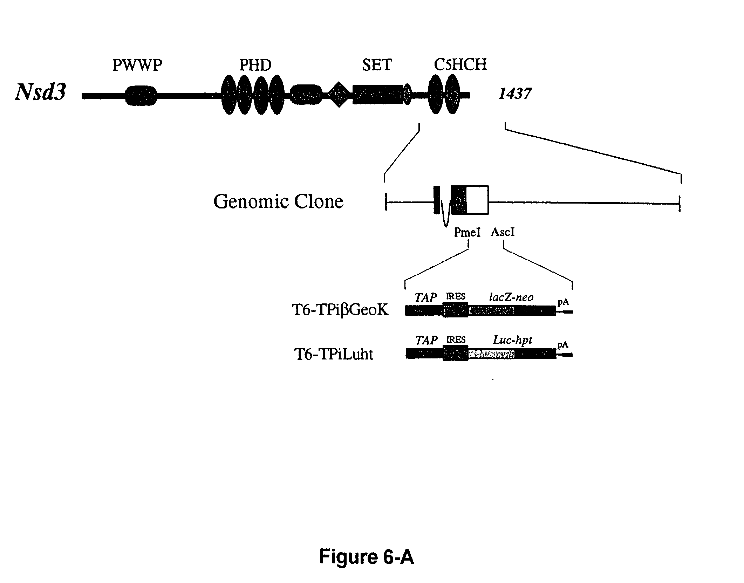

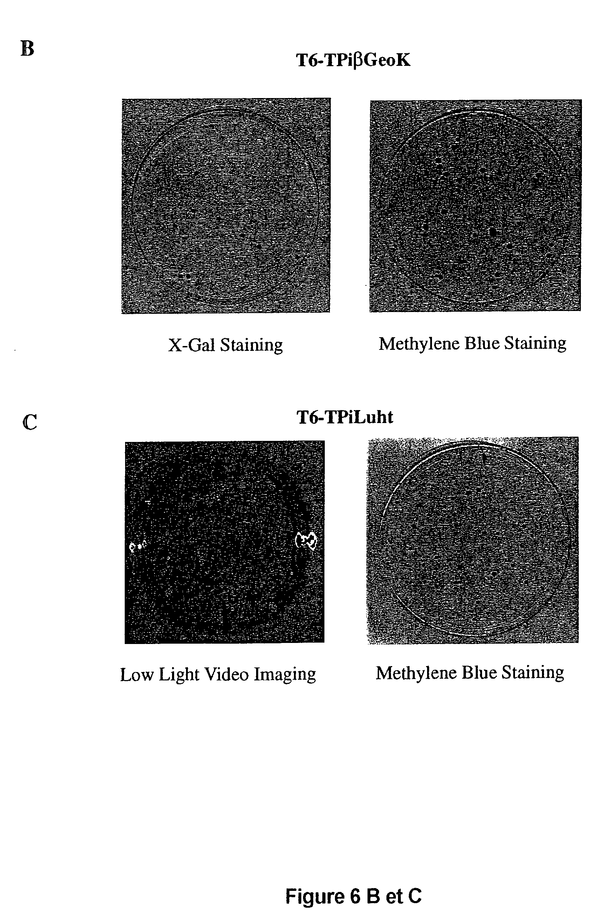

**Figure 6 : Comparison between LacZ- and luc-based vectors in a strategy designed to introduce a tag in the *Nsd3* locus.**
   **(A).** Schematic representation of the Nsd3 protein and its different domains. The genomic clone used contains the last two exons (exons 22 and 23) of the *Nsd3* gene, encoding the fifth PHD finger and the C5HCH motif. Targeting constructs were generated using ET recombination (Angrand *et al.*, 1999). The Nsd3 STOP codon was removed and two restriction sites introduced to clone a cassette in a second *in vitro* step. In T6-TPiβGeoK the cassette includes a promoterless *β-Geo* gene and in T6-TPiLuht, the cassette contains the promoterless Luc-hpt fusion gene.
   **(B).** The T6-TPiβGeoK construct was introduced into F9 cells and after G418-selection, recombinant candidates were visualized by X-Gal staining and the total number of clones by methylene blue staining of the same dish. **(C).** The T6-TPiLuht construct was introduced into F9 cells and after hygromycin B-selection, recombinant candidates were visualized by low light video imaging in presence of luciferin and the total number of clones by methylene blue staining of the same dish.

   This figure illustrates that both the *lacZ-neo* and the *luc-hpt* cassettes behave in a similar manner in terms of the total number of clones obtained and the frequency of candidate clones.

### EXAMPLES

The invention is illustrated by the results presented in the figures. The experiments were done according to the following Materials and Methods.

### MATERIALS and METHODS

DNA techniques. Large scale plasmid DNA preparation was performed with the Qiagen Plasmid Kit (Qiagen). Restriction endonucleases and T4 DNA ligase were purchased from New England Biolabs. Plasmids were grown in *Escherichia coli* strain XL1-blue [F'::Tn10 proA⁺B⁺ lacI^{q} Δ(lacZ)M15/recA1 endA1 gyrA96 (Nal^{r}) thi hsdR17 (rₖ⁻ mₖ⁺) supE44 relA1 lac].

PCR product preparation. All PCR reactions were performed in 50 µl reactions containing 5 units Amplitaq DNA polymerase (Perkin Elmer Cetus), 5 ng of plasmid and 1 pmol of each PCR primers for 30 cycles (94°C 30 sec, 55°C 1 min, 72°C 1 min). The chloramphenicol resistance gene was amplified from pMAK705 (Hashimoto-Gotoh and Sekiguchi, 1977) using the primers : 
5′TGGAGCAAGATCAGATGTAAATGGGAATCCAAGATAGTGGAGAAGAAGTAAAG
G GTCGAC CCC TGC CCT GAA CCG ACG ACC GGG T-3′ (SEQ ID
N°9), and
5′CTGTACTATTCAAACCTCACAGCCATGTTTGCAAACACTGACTTTGTGGGG
ACGCGT CGA ATA AAT ACC TGT GAC GGA AGA TG-3′ (SEQ ID
N°10),
 where the homology arms to Nsd3 [Angrand & al. (2001) *in press*] are in bold and the restriction sites for SalI and MluI are underlined. PCR products were purified using the Qiagen PCR Purification Kit and eluted with water, followed by digestion of any residual template DNA with DpnI. PCR products were then ethanol precipitated and resuspend in water (1 µg/µl).

Bacterial transformation. For ET recombination [Angrand *et al.*, 1999), the *Escherichia coli* strain JC8679 [Clark *et al*., 1984)[recB21, recC22, sbcA23, his-328, thr-1, ara-14, leuB6, Δ(gpt-proA)62, lacY1, tsx-33, glnV44(AS), galK2, rpsL31, kdgK51, xylA5, mtl-1, argE3(Oc), thi-1, Lam⁻, Rac⁺, Qsr¹⁺] was transformed by electroporation using a Bio-Rad Gene Pulser set at 2.5 kV, 200 Ω, and 25 µF. The transformed cells were suspended in 600 µl L-broth and incubated for 1 hour at 37°C before plating on L-agar containing zeocin (25 µg/ml) or chloramphenicol (100 µg/ml). Electroporationcompetent bacterial cells were made as follows: saturated, overnight JC8679 cultures were diluted 50-fold in L-broth, grown to an OD600 of 0.4 and chilled in ice for 30 min. Cells were centrifuged at 5,000 rpm for 15 min at -5°C. The pellet was resuspended in ice-cold 10% glycerol and centrifuged again (6,000 rpm,-5°C, 15 min). This was repeated twice more and the cell pellet was then suspended in 300 µl of ice-cold 10% glycerol. Aliquots (50 µl) were frozen in liquid nitrogen and stored at -80°C. For electroporation, cells were thawed on ice and added to 1 µl mix containing the vector DNA (0.5 µg) and the PCR products (0.5 µg).

F9 cell culture and transformation. F9 embryonic carcinoma (EC) cells were grown in Dulbecco's modified Eagle's medium supplemented with 10% fetal calf serum at 37°C in a humidity-saturated 7% CO₂ atmosphere. For transformation, F9 cells were trypsinized and resuspended in phosphate-buffered saline (PBS; 137 mM NaCl, 2.7 mM KCl, 4.3 mM Na₂HPO₄, 1.4 mM KH₂PO₄) at a concentration of 1 x 10⁷/ml. For each individual tranformation 0.8 ml cells (5.6 x 10⁷) were mixed with 15 µg linearized DNA in an electroporation cuvette with a 0.4-cm electrode gap. Cells were electroporated with a Bio-Rad Gene Pulser set at 200 V, 975 µF. The cells were plated onto four 10-cm gelatinized plates. After 24 hours, cells were fed with medium additionally supplemented with suitable antibiotics. Hygromycin B (Euromedex) was used at 250 µg/ml final concentration, and G418 (GIBCO BRL) at 400 µg/ml.

X-Gal Staining. Cells were fixed in PBS containing 1% formaldehyde (Merck), 0.2% glutaraldehyde (Sigma) and *lacZ* expression was detected by *in situ* X-Gal (5-bromo-4-chloro-3-β-D-galactopyranoside; Biomol) staining. Cells were incubated overnight in PBS with 5 mM potassium ferricyanide (Sigma), 5 mM potassium ferrocyanide (Sigma), 2 mM MgCl₂, 1 mg/ml X-Gal.

Methylene blue staining. The medium was removed from the dishes and cells were fixed *in situ* by addition of ethanol for 5 min. The solution was then replaced with 1% methylene blue in water for 10 min.

Detection of the *luciferase* activity. The *Photinus luciferase* activity was detected in the presence of 300 µM luciferin in PBS, and the *Renilla luciferase* activity was detected in presence of 2 µM coelenterazine (Molecular Probe) in PBS. The medium was removed from the dishes and *luciferase* activity monitored in presence of the corresponding substrate using a photon-counting camera (Hamamatsu) equipped with the Argus 50 software (Hamamatsu). Photon-counting was performed during 1 to 3 min.

Western blot analysis. Total cell extracts were obtained by freeze-thaw. Protein extracts were loaded onto 6% SDS-polyacrylamide gels, electrophoresed and blotted onto poly(vinylidene difluoride) (PVDF) membranes (Immobilon P; Millipore). Membranes were blocked with 5% non-fat milk in PBS, 0.1% Tween 20, and then incubated in 5% non-fat milk in PBS, 0.1% Tween 20 with peroxidase anti-peroxidase (PAP) antibodies (Sigma) for 1 hour. Detection was performed as described in the ECL Kit (Amersham).

### REFERENCES

Akiyama *et al.* (2000) Nucleic Acids Res., **28:** e77.

Angrand *et al.* (1999) Nucleic Acids Res., **27:** e16.

Angrand *et al.* (2001) *"NSD3*, a new SET domaincontaining gene, maps to 8p12 and is amplified in human breast cancer cell lines" *Genomics*, **74:** in *press.*

Arbones *et al.* (1994) Nature Genet. **6:** 90-97.

Brown *et al.* (1997) Science **277:** 831-834.

Capecchi (1989) Science **244:** 1288-1292.

Charbonneau *et al.* (1979) J. Biol. Chem. **254:**769-780.

Clark et *al.* (1984) *Cold Spring Harb. Symp. Quant. Biol.*, **49:** 453-462.

Collier *et al.* (1998) J. Gen. Virol. **79:** 2359-2366.

Cormier *et al.* (1973) J. Cell. Physiol. **81:**291-298.

Cormier (1981) Bioluminescence and Chemiluminescence, pp. 225-233.

De Wet *et al.* (1985) Proc. Natl. Acad. Sci. USA **82:**7870-7873.

De Wet et *al.* (1986) Methods in Enzymology **133:**3-14.

Friedrich *et al.* (1991) Genes Dev. 5: 1513-1523.

Hanson *et al.* (1995) Mol. Cell. Biol. **15:** 45-51.

Hashimoto-Gotoh and Sekiguchi (1977) *J. Bacteriol.*, **131:** 405-412.

Hastings (1995) Cell Physiology : Source Book, N. Sperelakis (Ed.), Academic Press, pp. 665-681.

Hori *et al.* (1977) Proc. Natl. Acad. Sci. USA **74:**4285-4287.

Huez *et al.* (1998) Mol. Cell. Biol. **18:** 6178-6190.

Inouye *et al.* (1975) Jap. Soc. Chem. Lett. 141-144.

Jasin *et al.* (1988) Genes Dev. **2:** 1353-1363.

Langridge *et al.* (1996) J. Virol. Methods **61:** 151-156.

Leach *et al.* (1986) Methods in Enzymology **133:**51-70, esp. Table 1.

Lopez de Quinto et Martinez-Salas (1999) J. Virol. **71:** 4171-4175.

Lorenz *et al.* (1981) Proc. Natl. Acad. Sci. USA **88:**4438-4442.

Lorenz *et al.* (1996) J. Biolumin. Chemilumin. **11:** 31-37.

Mansour *et al.* (1988) Nature, **336:** 348-352.

Matthews *et al.* (1979) Biochemistry **16:** 85-91.

Mountford and Smith (1995) Trends Genet. **11:** 179-184.

Neddleman and Wunsch (1970) J. Mol. Biol. **48:** 443.

Pearson and Lipman (1988) Proc. Natl. Acad. Sci. USA **85:** 2444-2445.

Pelletier and Sonengerg (1988) Nature **334:** 320-325.

Prasher *et al.* (1985) Biochem. Biophys. Res. Commun. **126:** 1259-1268.

Rigaut *et al.* (1999) Nature Biotech. **17:** 1030-1032.

Sambrook *et al.* (1989) Molecular cloning : a laboratory manual second edition, Cold Spring Harbor Laboratory Press. Cold Spring Harbor, NY. USA.

Sedivy *et al.* (1989) Proc. Natl. Acad. Sci. USA **86:** 227-231.

Sedivy *et al.* (1999) *Science*, **283:** (Abstract 293/5398/9a)

Smith and Waterman (1981) Ad. App. Math. 2: 482.

Storck *et al.* (1996) Nucleic Acids Res. **24:** 4594-4596.

Waldman (1992) Crit. Rev. Oncol. Hematol. **12:** 49-64.

Ward *et al.* (1975) Proc. Natl. Acad. Sci. USA **72:** 2530-2534.

Ward *et al.* (1979) J. Biol. Chem. **254:** 781-788.

Watson *et al.* (1987) Molecular Biology of the Gene ; 4^{th} Edition, The Benjamin/Cummings Pub. Co., p. 224).

Wienhausen *et al.* (1985) Photochemistry and Photobiology **42:** 609-611.

Wiles *et al.* (2000) Nature Genet. **24:** 13-14.

Wilson and Hastings (1998) Bioluminescence. Annu. Rev. Cell Dev. Biol. **14:** 197-230.

Yañez *et al.* (1998) Gene Therapy **5:** 149-159.

Yang *et al.* (1997) Nature Biotech. **15:** 859-865.

Zambrowicz *et al.* (1998) Nature **392:** 608-611.

## Claims

1. Active recombinant fusion protein comprising a *luciferase*, or a fragment, or a variant thereof fused to a selection marker protein, or a fragment or a variant thereof, said fusion protein having at least a light-producing activity and a selection marker activity.

2. Fusion protein according to claim 1, wherein said *luciferase* is isolated from an organism selected in the group consisting of bacteria, mushrooms, Dinoflagellates, Cnidaria (coelenterates), annelids, crustacea, insects, Echinoderms, Chordates, fishes.

3. Fusion protein according to claim 2, wherein said *luciferase* isolated from Cnidaria is selected in the group consisting of jellyfish, hydroid, sea pansy, ctenophores.

4. Fusion protein according to claim 3, wherein said *luciferase* isolated from sea pansy is *Renilla reniformis luciferase.*

5. Fusion protein according to claim 2, wherein said *luciferase* isolated from insects is selected in the group consisting of Coleopterids (beetles), firefly, click beetles, railroad worm, diptera (flies).

6. Fusion protein according to claim 5, wherein said *luciferase* isolated from firefly is *Photinus luciferase.*

7. Fusion protein according to claims 1 to 6, wherein said selection marker protein is a positive selection marker protein encoded by a gene selected in the group consisting of antibiotic resistance genes, hisD genes, *Hypoxanthine phosphoribosyl transferase* (HPRT) genes, guanine-phosphoribosyl-transferase (Gpt) genes.

8. Fusion protein according to claim 7, wherein said antibiotic resistance gene is selected in the group consisting of hygromycin resistance genes, neomycin resistance genes, tetracyclin resistance genes, ampicillin resistance genes, kanamycin resistance genes, phleomycin resistance genes, bleomycin resistance genes, geneticin resistance genes, carbenicillin resistance genes, chloramphenicol resistance genes, puromycin resistance genes, blasticidin-S-deaminase genes.

9. Fusion protein according to claim 8, wherein said antibiotic resistance gene is a hygromycin resistance gene.

10. Fusion protein according to claim 9, wherein said hygromycin resistance gene is an *Escherichia coli* hygromycin-B phosphotransferase (hpt) gene.

11. Fusion protein according to claim 10, comprising *Photinus luciferase* (Luc) or a fragment or variant thereof, fused to said *Escherichia coli* Hygromycin-B phosphotransferase (hpt) gene or a fragment or variant thereof.

12. Fusion protein according to claim 11 named *Luht*, wherein the sequence of said fusion protein is SEQ ID N° 2.

13. Fusion protein according to claim 10, comprising *Renilla reniformis luciferase* (Ruc) or a fragment or variant thereof, fused to said *Escherichia coli* hygromycin B phosphotransferase (hpt) gene or a fragment or variant thereof.

14. Fusion protein according to claim 13 named *Ruht,* wherein the sequence of said fusion protein is SEQ ID No. 4.

15. Fusion protein according to claim 8, wherein said antibiotic resistance gene is a neomycin resistance gene.

16. Fusion protein according to claim 15, wherein said neomycin resistance gene is the Tn5 neomycin resistance gene.

17. Fusion protein according to claim 16 comprising *Photinus luciferase* (Luc) or a fragment or variant thereof, fused to said Tn5 neomycin resistance gene or a fragment or variant thereof.

18. Fusion protein according to claim 17 named *LeoK*, wherein the sequence of said fusion protein is SEQ ID No. 6.

19. Fusion protein according to claim 16 comprising *Renilla reniformis luciferase* (Ruc) or a fragment or variant thereof, fused to said Tn5 neomycin resistance gene or a fragment or variant thereof.

20. Fusion protein according to claim 19 named *ReoK*, wherein the sequence of said fusion protein is SEQ ID No. 8.

21. Recombinant polynucleotide encoding for a fusion protein according to claims 1 to 17.

22. Recombinant polynucleotide according to claim 21 with sequence SEQ ID No. 1 encoding for the fusion protein according to claim 12.

23. Recombinant polynucleotide according to claim 21 with sequence SEQ ID No. 3 encoding for the fusion protein according to claim 14.

24. Recombinant polynucleotide according to claim 21 with sequence SEQ ID No. 5 encoding for the fusion protein according to claim 18.

25. Recombinant polynucleotide according to claim 21 with sequence SEQ ID No. 7 encoding for the fusion protein according to claim 20.

26. Recombinant polynucleotide comprising a polynucleotide selected among :
a) the polynucleotides of sequences SEQ ID No. 1, SEQ ID No. 3, SEQ ID No. 5, SEQ ID No. 7 ;
b) the polynucleotides presenting at least 80% identity after optimal alignment with the polynucleotide of step a) ;
c) complementary sequence or RNA sequence corresponding to a polynucleotide of step a) or b).

27. A DNA cassette comprising a promoter operably linked to a polynucleotide according to claims 19 to 24.

28. A vector comprising a polynucleotide according to claims 21 to 26.

29. An expression vector comprising at least one DNA cassette according to claim 27.

30. A gene targeting vector comprising at least the following nucleotides sequences in the following order :
(i) a first sequence homologous to the targeted gene ;
(ii) optionally a genetic element ;
(iii) an internal ribosome entry site (IRES) ;
(iv) a polynucleotide according to claims 21 to 26 or a DNA cassette according to claim 27 ;
(v) a polyadenylation site ;
(vi) a second sequence homologous to the targeted gene.

31. A gene trap vector comprising at least the following nucleotide sequences in the following order :
(i) a genetic element ;
(ii) an internal ribosome-entry site (IRES) ;
(iii) a polynucleotide according to claims 21 to 26 ;
(iv) optionally, a polyadenylation site.

32. A gene trap vector comprising at least the following nucleotide sequences in the following order :
(i) a promoter ;
(ii) an internal ribosome-entry site (IRES) ;
(iii) a polynucleotide according to claims 21 to 26 ;
(iv) optionally, a polyadenylation site.

33. A gene trap vector comprising at least the following nucleotide sequences in the following order :
(i) an acceptor splicing site;
(ii) an internal ribosome-entry site (IRES) ;
(iii) a polynucleotide according to claims 21 to 26 ;
(iv) a donor splicing site.

34. A host cell transformed by at least one polynucleotide according to claims 21 to 26, one DNA cassette according to claim 27, or one vector according to claims 28 to 33.

35. Cell according to claim 34 wherein the protein fusion encoded by said polynucleotide according to claims 21 to 26, DNA cassette according to claim 27, or vector according to claims 28 to 33, is expressed and biologically active in said cell.

36. Cell according to claims 34 to 35 wherein said cell is selected among eukaryotic and procaryotic cells.

37. Eukaryotic cell according to claim 36 wherein said cells is selected among human cells, murine cells, yeast cells.

38. Animal, excepted humans, comprising at least a cell according to claims 34 to 37.

39. Animal according to claim 38 wherein said animal is a mouse.

40. *In vitro* method of selecting cells into the genome of which a genetic element has been targeted into at least one predefined locus, said method comprising the steps of :
a) introducing a vector or a fragment thereof according to claims 28 to 30 into a host cell ;
b) culturing said cell in the presence of an appropriate selective agent under conditions whereby said vector or fragment thereof integrates by homologous recombination into at least one predefined locus of the genome of said cell, and said cell expresses the fusion protein encoded by said vector or a fragment thereof ;
c) contacting cells with appropriate luciferin ;
d) identifying cells expressing the *luciferase* activity of said fusion protein by photon emission detection, then selecting cells.

41. *In vitro* method for trapping unknown gene, said method comprising the steps of :
a) introducing a vector or a fragment thereof according to claims 28, 29, 31, 32 and 33 into a host cell ;
b) culturing said cell in the presence of an appropriate selective agent under conditions whereby said vector or fragment thereof integrates into the genome of said cells, said cells express the fusion protein encoded by said vector or a fragment thereof ;
c) contacting cells with luciferin ;
d) identifying cells expressing *luciferase* activity of said fusion protein by photon emission detection, then selecting cells ; and
e) isolation and/or identification of the gene(s) in which the trapping vector integrates.

42. Method according to claim 41, wherein the *luciferase* activity is further quantified to estimate the level of expression of said unknown gene in said cell.

43. Kit for transgenic cell selection and/or for monitoring gene expression comprising :
a) compound selected in the group consisting of :
. a polynucleotide according to claims 21 to 26,
. a vector according to claims 28 to 33,
. a DNA cassette according to claim 27 ;
b) at least one luciferin, which is a substrate for the *luciferase* activity of the fusion protein encoded by said compound ;
c) at least one appropriate selective agent.

44. Use of a fusion protein according to claims 1 to 20, or a polynucleotide according to claims 21 to 26, or a vector according to claims 28 to 33, or a DNA cassette according to claim 27, or a kit according to claim 43, for transgenic cell selection and/or monitoring gene expression.

45. Use according to claim 44 to perform gene targeting, gene tagging, gene knock-out (KO), gene knock-in (KI), polyA trap, exon trap, promoter trap.


SEQUENCE LISTING

<110> Association pour le développement de la recherche en génétique
      moléculaire ADEREGEM

<120> Luciferase-selection marker fusion proteins, and polynucleotides
      encoding such proteins, and research applications

<130> D19378

<160> 10

<170> PatentIn Ver. 2.1

<210> 1
<211> 2695
<212> DNA
<213> Artificial Sequence

<220>
<223> Description of Artificial Sequence: LUHT

<220>
<221> CDS
<222> (3)..(2681)

<400> 1
cc atg gaa gac gcc aaa aac ata aag aaa ggc ccg gcg cca ttc tat     47
   Met Glu Asp Ala Lys Asn Ile Lys Lys Gly Pro Ala Pro Phe Tyr
     1               5                  10                  15

ccg ctg gaa gat gga acc gct gga gag caa ctg cat aag gct atg aag     95
Pro Leu Glu Asp Gly Thr Ala Gly Glu Gln Leu His Lys Ala Met Lys
                 20                  25                  30

aga tac gcc ctg gtt cct gga aca att gct ttt aca gat gca cat atc    143
Arg Tyr Ala Leu Val Pro Gly Thr Ile Ala Phe Thr Asp Ala His Ile
             35                  40                  45

gag gtg gac atc act tac gct gag tac ttc gaa atg tcc gtt cgg ttg    191
Glu Val Asp Ile Thr Tyr Ala Glu Tyr Phe Glu Met Ser Val Arg Leu
         50                  55                  60

gca gaa gct atg aaa cga tat ggg ctg aat aca aat cac aga atc gtc    239
Ala Glu Ala Met Lys Arg Tyr Gly Leu Asn Thr Asn His Arg Ile Val
     65                  70                  75

gta tgc agt gaa aac tct ctt caa ttc ttt atg ccg gtg ttg ggc gcg    287
Val Cys Ser Glu Asn Ser Leu Gln Phe Phe Met Pro Val Leu Gly Ala
 80                  85                  90                  95

tta ttt atc gga gtt gca gtt gcg ccc gcg aac gac att tat aat gaa    335
Leu Phe Ile Gly Val Ala Val Ala Pro Ala Asn Asp Ile Tyr Asn Glu
                100                 105                 110

cgt gaa ttg ctc aac agt atg ggc att tcg cag cct acc gtg gtg ttc    383
Arg Glu Leu Leu Asn Ser Met Gly Ile Ser Gln Pro Thr Val Val Phe
            115                 120                 125

gtt tcc aaa aag ggg ttg caa aaa att ttg aac gtg caa aaa aag ctc    431
Val Ser Lys Lys Gly Leu Gln Lys Ile Leu Asn Val Gln Lys Lys Leu

130                 135                 140

cca atc atc caa aaa att att atc atg gat tct aaa acg gat tac cag     479
Pro Ile Ile Gln Lys Ile Ile Ile Met Asp Ser Lys Thr Asp Tyr Gln
    145                 150                 155

gga ttt cag tcg atg tac acg ttc gtc aca tct cat cta cct ccc ggt     527
Gly Phe Gln Ser Met Tyr Thr Phe Val Thr Ser His Leu Pro Pro Gly
160                 165                 170                 175

ttt aat gaa tac gat ttt gtg cca gag tcc ttc gat agg gac aag aca     575
Phe Asn Glu Tyr Asp Phe Val Pro Glu Ser Phe Asp Arg Asp Lys Thr
                180                 185                 190

att gca ctg atc atg aac tcc tct gga tct act ggt ctg cct aaa ggt     623
Ile Ala Leu Ile Met Asn Ser Ser Gly Ser Thr Gly Leu Pro Lys Gly
            195                 200                 205

gtc gct ctg cct cat aga act gcc tgc gtg aga ttc tcg cat gcc aga     671
Val Ala Leu Pro His Arg Thr Ala Cys Val Arg Phe Ser His Ala Arg
        210                 215                 220

gat cct att ttt ggc aat caa atc att ccg gat act gcg att tta agt     719
Asp Pro Ile Phe Gly Asn Gln Ile Ile Pro Asp Thr Ala Ile Leu Ser
    225                 230                 235

gtt gtt cca ttc cat cac ggt ttt gga atg ttt act aca ctc gga tat     767
Val Val Pro Phe His His Gly Phe Gly Met Phe Thr Thr Leu Gly Tyr
240                 245                 250                 255

ttg ata tgt gga ttt cga gtc gtc tta atg tat aga ttt gaa gaa gag     815
Leu Ile Cys Gly Phe Arg Val Val Leu Met Tyr Arg Phe Glu Glu Glu
                260                 265                 270

ctg ttt ctg agg agc ctt cag gat tac aag att caa agt gcg ctg ctg     863
Leu Phe Leu Arg Ser Leu Gln Asp Tyr Lys Ile Gln Ser Ala Leu Leu
            275                 280                 285

gtg cca acc cta ttc tcc ttc ttc gcc aaa agc act ctg att gac aaa     911
Val Pro Thr Leu Phe Ser Phe Phe Ala Lys Ser Thr Leu Ile Asp Lys
        290                 295                 300

tac gat tta tct aat tta cac gaa att gct tct ggt ggc gct ccc ctc     959
Tyr Asp Leu Ser Asn Leu His Glu Ile Ala Ser Gly Gly Ala Pro Leu
    305                 310                 315

tct aag gaa gtc ggg gaa gcg gtt gcc aag agg ttc cat ctg cca ggt     1007
Ser Lys Glu Val Gly Glu Ala Val Ala Lys Arg Phe His Leu Pro Gly
320                 325                 330                 335

atc agg caa gga tat ggg ctc act gag act aca tca gct att ctg att     1055
Ile Arg Gln Gly Tyr Gly Leu Thr Glu Thr Thr Ser Ala Ile Leu Ile
                340                 345                 350

aca ccc gag ggg gat gat aaa ccg ggc gcg gtc ggt aaa gtt gtt cca     1103
Thr Pro Glu Gly Asp Asp Lys Pro Gly Ala Val Gly Lys Val Val Pro
            355                 360                 365

ttt ttt gaa gcg aag gtt gtg gat ctg gat acc ggg aaa acg ctg ggc     1151
Phe Phe Glu Ala Lys Val Val Asp Leu Asp Thr Gly Lys Thr Leu Gly
        370                 375                 380

gtt aat caa aga ggc gaa ctg tgt gtg aga ggt cct atg att atg tcc 1199
Val Asn Gln Arg Gly Glu Leu Cys Val Arg Gly Pro Met Ile Met Ser
    385                 390                 395

ggt tat gta aac aat ccg gaa gcg acc aac gcc ttg att gac aag gat 1247
Gly Tyr Val Asn Asn Pro Glu Ala Thr Asn Ala Leu Ile Asp Lys Asp
400                 405                 410                 415

gga tgg cta cat tct gga gac ata gct tac tgg gac gaa gac gaa cac 1295
Gly Trp Leu His Ser Gly Asp Ile Ala Tyr Trp Asp Glu Asp Glu His
                420                 425                 430

ttc ttc atc gtt gac cgc ctg aag tct ctg att aag tac aaa ggc tat 1343
Phe Phe Ile Val Asp Arg Leu Lys Ser Leu Ile Lys Tyr Lys Gly Tyr
            435                 440                 445

cag gtg gct ccc gct gaa ttg gaa tcc atc ttg ctc caa cac ccc aac 1391
Gln Val Ala Pro Ala Glu Leu Glu Ser Ile Leu Leu Gln His Pro Asn
        450                 455                 460

atc ttc gac gca ggt gtc gca ggt ctt ccc gac gat gac gcc ggt gaa 1439
Ile Phe Asp Ala Gly Val Ala Gly Leu Pro Asp Asp Asp Ala Gly Glu
    465                 470                 475

ctt ccc gcc gcc gtt gtt gtt ttg gag cac gga aag acg atg acg gaa 1487
Leu Pro Ala Ala Val Val Val Leu Glu His Gly Lys Thr Met Thr Glu
480                 485                 490                 495

aaa gag atc gtg gat tac gtc gcc agt caa gta aca acc gcg aaa aag 1535
Lys Glu Ile Val Asp Tyr Val Ala Ser Gln Val Thr Thr Ala Lys Lys
                500                 505                 510

ttg cgc gga gga gtt gtg ttt gtg gac gaa gta ccg aaa ggt ctt acc 1583
Leu Arg Gly Gly Val Val Phe Val Asp Glu Val Pro Lys Gly Leu Thr
            515                 520                 525

gga aaa ctc gac gca aga aaa atc aga gag atc ctc ata aag gcc aag 1631
Gly Lys Leu Asp Ala Arg Lys Ile Arg Glu Ile Leu Ile Lys Ala Lys
        530                 535                 540

aag ggc gga aag atc gcc gtg atg cat atg aaa aag cct gaa ctc acc 1679
Lys Gly Gly Lys Ile Ala Val Met His Met Lys Lys Pro Glu Leu Thr
    545                 550                 555

gcg acg tct gtc gag aag ttt ctg atc gaa aag ttc gac agc gtc tcc 1727
Ala Thr Ser Val Glu Lys Phe Leu Ile Glu Lys Phe Asp Ser Val Ser
560                 565                 570                 575

gac ctg atg cag ctc tcg gag ggc gaa gaa tct cgt gct ttc agc ttc 1775
Asp Leu Met Gln Leu Ser Glu Gly Glu Glu Ser Arg Ala Phe Ser Phe
                580                 585                 590

gat gta gga ggg cgt gga tat gtc ctg cgg gta aat agc tgc gcc gat 1823
Asp Val Gly Gly Arg Gly Tyr Val Leu Arg Val Asn Ser Cys Ala Asp
            595                 600                 605

ggt ttc tac aaa gat cgt tat gtt tat cgg cac ttt gca tcg gcc gcg 1871
Gly Phe Tyr Lys Asp Arg Tyr Val Tyr Arg His Phe Ala Ser Ala Ala
        610                 615                 620

ctc ccg att ccg gaa gtg ctt gac att ggg gaa ttc agc gag agc ctg 1919
Leu Pro Ile Pro Glu Val Leu Asp Ile Gly Glu Phe Ser Glu Ser Leu
    625                 630                 635

acc tat tgc atc tcc cgc cgt gca cag ggt gtc acg ttg caa gac ctg 1967
Thr Tyr Cys Ile Ser Arg Arg Ala Gln Gly Val Thr Leu Gln Asp Leu
640                 645                 650                 655

cct gaa acc gaa ctg ccc gct gtt ctg cag ccg gtc gcg gag gcc atg 2015
Pro Glu Thr Glu Leu Pro Ala Val Leu Gln Pro Val Ala Glu Ala Met
                660                 665                 670

gat gcg atc gct gcg gcc gat ctt agc cag acg agc ggg ttc ggc cca 2063
Asp Ala Ile Ala Ala Ala Asp Leu Ser Gln Thr Ser Gly Phe Gly Pro
            675                 680                 685

ttc gga ccg caa gga atc ggt caa tac act aca tgg cgt gat ttc ata 2111
Phe Gly Pro Gln Gly Ile Gly Gln Tyr Thr Thr Trp Arg Asp Phe Ile
        690                 695                 700

tgc gcg att gct gat ccc cat gtg tat cac tgg caa act gtg atg gac 2159
Cys Ala Ile Ala Asp Pro His Val Tyr His Trp Gln Thr Val Met Asp
    705                 710                 715

gac acc gtc agt gcg tcc gtc gcg cag gct ctc gat gag ctg atg ctt 2207
Asp Thr Val Ser Ala Ser Val Ala Gln Ala Leu Asp Glu Leu Met Leu
720                 725                 730                 735

tgg gcc gag gac tgc ccc gaa gtc cgg cac ctc gtg cac gcg gat ttc 2255
Trp Ala Glu Asp Cys Pro Glu Val Arg His Leu Val His Ala Asp Phe
                740                 745                 750

ggc tcc aac aat gtc ctg acg gac aat ggc cgc ata aca gcg gtc att 2303
Gly Ser Asn Asn Val Leu Thr Asp Asn Gly Arg Ile Thr Ala Val Ile
            755                 760                 765

gac tgg agc gag gcg atg ttc ggg gat tcc caa tac gag gtc gcc aac 2351
Asp Trp Ser Glu Ala Met Phe Gly Asp Ser Gln Tyr Glu Val Ala Asn
        770                 775                 780

atc ttc ttc tgg agg ccg tgg ttg gct tgt atg gag cag cag acg cgc 2399
Ile Phe Phe Trp Arg Pro Trp Leu Ala Cys Met Glu Gln Gln Thr Arg
    785                 790                 795

tac ttc gag cgg agg cat ccg gag ctt gca gga tcg ccg cgg ctc cgg 2447
Tyr Phe Glu Arg Arg His Pro Glu Leu Ala Gly Ser Pro Arg Leu Arg
800                 805                 810                 815

gcg tat atg ctc cgc att ggt ctt gac caa ctc tat cag agc ttg gtt 2495
Ala Tyr Met Leu Arg Ile Gly Leu Asp Gln Leu Tyr Gln Ser Leu Val
                820                 825                 830

gac ggc aat ttc gat gat gca gct tgg gcg cag ggt cga tgc gac gca 2543
Asp Gly Asn Phe Asp Asp Ala Ala Trp Ala Gln Gly Arg Cys Asp Ala
            835                 840                 845

atc gtc cga tcc gga gcc ggg act gtc ggg cgt aca caa atc gcc cgc 2591
Ile Val Arg Ser Gly Ala Gly Thr Val Gly Arg Thr Gln Ile Ala Arg
        850                 855                 860

aga agc gcg gcc gtc tgg acc gat ggc tgt gta gaa gta ctc gcc gat 2639

Arg Ser Ala Ala Val Trp Thr Asp Gly Cys Val Glu Val Leu Ala Asp
    865                 870                 875

agt gga aac cga cgc ccc agc act cgt ccg agg gca aag gaa          2681
Ser Gly Asn Arg Arg Pro Ser Thr Arg Pro Arg Ala Lys Glu
880                 885                 890

tagcacacta gtat                                                  2695

<210> 2
<211> 893
<212> PRT
<213> Artificial Sequence
<223> Description of Artificial Sequence: LUHT

<400> 2
Met Glu Asp Ala Lys Asn Ile Lys Lys Gly Pro Ala Pro Phe Tyr Pro
  1               5                  10                  15

Leu Glu Asp Gly Thr Ala Gly Glu Gln Leu His Lys Ala Met Lys Arg
             20                  25                  30

Tyr Ala Leu Val Pro Gly Thr Ile Ala Phe Thr Asp Ala His Ile Glu
         35                  40                  45

Val Asp Ile Thr Tyr Ala Glu Tyr Phe Glu Met Ser Val Arg Leu Ala
     50                  55                  60

Glu Ala Met Lys Arg Tyr Gly Leu Asn Thr Asn His Arg Ile Val Val
 65                  70                  75                  80

Cys Ser Glu Asn Ser Leu Gln Phe Phe Met Pro Val Leu Gly Ala Leu
                 85                  90                  95

Phe Ile Gly Val Ala Val Ala Pro Ala Asn Asp Ile Tyr Asn Glu Arg
            100                 105                 110

Glu Leu Leu Asn Ser Met Gly Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Leu Pro Lys Gly Val
        195                 200                 205

Ala Leu Pro His Arg Thr Ala Cys Val Arg Phe Ser His Ala Arg Asp
    210                 215                 220

Pro Ile Phe Gly Asn Gln Ile Ile Pro Asp Thr Ala Ile Leu Ser Val
225                 230                 235                 240

Val Pro Phe His His Gly Phe Gly Met Phe Thr Thr Leu Gly Tyr Leu
                245                 250                 255

Ile Cys Gly Phe Arg Val Val Leu Met Tyr Arg Phe Glu Glu Glu Leu
            260                 265                 270

Phe Leu Arg Ser Leu Gln Asp Tyr Lys Ile Gln Ser Ala Leu Leu Val
        275                 280                 285

Pro Thr Leu Phe Ser Phe Phe Ala Lys Ser Thr Leu Ile Asp Lys Tyr
    290                 295                 300

Asp Leu Ser Asn Leu His Glu Ile Ala Ser Gly Gly Ala Pro Leu Ser
305                 310                 315                 320

Lys Glu Val Gly Glu Ala Val Ala Lys Arg Phe His Leu Pro Gly Ile
                325                 330                 335

Arg Gln Gly Tyr Gly Leu Thr Glu Thr Thr Ser Ala Ile Leu Ile Thr
            340                 345                 350

Pro Glu Gly Asp Asp Lys Pro Gly Ala Val Gly Lys Val Val Pro Phe
        355                 360                 365

Phe Glu Ala Lys Val Val Asp Leu Asp Thr Gly Lys Thr Leu Gly Val
    370                 375                 380

Asn Gln Arg Gly Glu Leu Cys Val Arg Gly Pro Met Ile Met Ser Gly
385                 390                 395                 400

Tyr Val Asn Asn Pro Glu Ala Thr Asn Ala Leu Ile Asp Lys Asp Gly
                405                 410                 415

Trp Leu His Ser Gly Asp Ile Ala Tyr Trp Asp Glu Asp Glu His Phe
            420                 425                 430

Phe Ile Val Asp Arg Leu Lys Ser Leu Ile Lys Tyr Lys Gly Tyr Gln
        435                 440                 445

Val Ala Pro Ala Glu Leu Glu Ser Ile Leu Leu Gln His Pro Asn Ile
    450                 455                 460

Phe Asp Ala Gly Val Ala Gly Leu Pro Asp Asp Asp Ala Gly Glu Leu
465                 470                 475                 480

Pro Ala Ala Val Val Val Leu Glu His Gly Lys Thr Met Thr Glu Lys
                485                 490                 495

Glu Ile Val Asp Tyr Val Ala Ser Gln Val Thr Thr Ala Lys Lys Leu
            500                 505                 510

Arg Gly Gly Val Val Phe Val Asp Glu Val Pro Lys Gly Leu Thr Gly
        515                 520                 525

Lys Leu Asp Ala Arg Lys Ile Arg Glu Ile Leu Ile Lys Ala Lys Lys
    530                 535                 540

Gly Gly Lys Ile Ala Val Met His Met Lys Lys Pro Glu Leu Thr Ala
545                 550                 555                 560

Thr Ser Val Glu Lys Phe Leu Ile Glu Lys Phe Asp Ser Val Ser Asp
                565                 570                 575

Leu Met Gln Leu Ser Glu Gly Glu Glu Ser Arg Ala Phe Ser Phe Asp
            580                 585                 590

Val Gly Gly Arg Gly Tyr Val Leu Arg Val Asn Ser Cys Ala Asp Gly
        595                 600                 605

Phe Tyr Lys Asp Arg Tyr Val Tyr Arg His Phe Ala Ser Ala Ala Leu
    610                 615                 620

Pro Ile Pro Glu Val Leu Asp Ile Gly Glu Phe Ser Glu Ser Leu Thr
625                 630                 635                 640

Tyr Cys Ile Ser Arg Arg Ala Gln Gly Val Thr Leu Gln Asp Leu Pro
                645                 650                 655

Glu Thr Glu Leu Pro Ala Val Leu Gln Pro Val Ala Glu Ala Met Asp
            660                 665                 670

Ala Ile Ala Ala Ala Asp Leu Ser Gln Thr Ser Gly Phe Gly Pro Phe
        675                 680                 685

Gly Pro Gln Gly Ile Gly Gln Tyr Thr Thr Trp Arg Asp Phe Ile Cys
    690                 695                 700

Ala Ile Ala Asp Pro His Val Tyr His Trp Gln Thr Val Met Asp Asp
705                 710                 715                 720

Thr Val Ser Ala Ser Val Ala Gln Ala Leu Asp Glu Leu Met Leu Trp
                725                 730                 735

Ala Glu Asp Cys Pro Glu Val Arg His Leu Val His Ala Asp Phe Gly
            740                 745                 750

Ser Asn Asn Val Leu Thr Asp Asn Gly Arg Ile Thr Ala Val Ile Asp
        755                 760                 765

Trp Ser Glu Ala Met Phe Gly Asp Ser Gln Tyr Glu Val Ala Asn Ile
    770                 775                 780

Phe Phe Trp Arg Pro Trp Leu Ala Cys Met Glu Gln Gln Thr Arg Tyr
785                 790                 795                 800

Phe Glu Arg Arg His Pro Glu Leu Ala Gly Ser Pro Arg Leu Arg Ala
                805                 810                 815

Tyr Met Leu Arg Ile Gly Leu Asp Gln Leu Tyr Gln Ser Leu Val Asp
            820                 825                 830

Gly Asn Phe Asp Asp Ala Ala Trp Ala Gln Gly Arg Cys Asp Ala Ile
        835                 840                 845

Val Arg Ser Gly Ala Gly Thr Val Gly Arg Thr Gln Ile Ala Arg Arg
    850                 855                 860

Ser Ala Ala Val Trp Thr Asp Gly Cys Val Glu Val Leu Ala Asp Ser
865                 870                 875                 880

Gly Asn Arg Arg Pro Ser Thr Arg Pro Arg Ala Lys Glu
                885                 890

<210> 3
<211> 1982
<212> DNA
<213> Artificial Sequence

<220>
<223> Description of Artificial Sequence: RUHT

<220>
<221> CDS
<222> (7)..(1968)

<400> 3
atggtc atg act tcg aaa gtt tat gat cca gaa caa agg aaa cgg atg      48
       Met Thr Ser Lys Val Tyr Asp Pro Glu Gln Arg Lys Arg Met
         1               5                  10

ata act ggt ccg cag tgg tgg gcc aga tgt aaa caa atg aat gtt ctt      96
Ile Thr Gly Pro Gln Trp Trp Ala Arg Cys Lys Gln Met Asn Val Leu
 15                  20                  25                  30

gat tca ttt att aat tat tat gat tca gaa aaa cat gca gaa aat gct     144
Asp Ser Phe Ile Asn Tyr Tyr Asp Ser Glu Lys His Ala Glu Asn Ala
                 35                  40                  45

gtt att ttt tta cat ggt aac gcg gcc tct tct tat tta tgg cga cat     192
Val Ile Phe Leu His Gly Asn Ala Ala Ser Ser Tyr Leu Trp Arg His
             50                  55                  60

gtt gtg cca cat att gag cca gta gcg cgg tgt att ata cca gac ctt     240
Val Val Pro His Ile Glu Pro Val Ala Arg Cys Ile Ile Pro Asp Leu
         65                  70                  75

att ggt atg ggc aaa tca ggc aaa tct ggt aat ggt tct tat agg tta     288
Ile Gly Met Gly Lys Ser Gly Lys Ser Gly Asn Gly Ser Tyr Arg Leu
     80                  85                  90

ctt gat cat tac aaa tat ctt act gca tgg ttt gaa ctt ctt aat tta     336
Leu Asp His Tyr Lys Tyr Leu Thr Ala Trp Phe Glu Leu Leu Asn Leu
 95                 100                 105                 110

cca aag aag atc att ttt gtc ggc cat gat tgg ggt gct tgt ttg gca     384
Pro Lys Lys Ile Ile Phe Val Gly His Asp Trp Gly Ala Cys Leu Ala
                115                 120                 125

ttt cat tat agc tat gag cat caa gat aag atc aaa gca ata gtt cac     432
Phe His Tyr Ser Tyr Glu His Gln Asp Lys Ile Lys Ala Ile Val His
            130                 135                 140

gct gaa agt gta gta gat gtg att gaa tca tgg gat gaa tgg cct gat     480
Ala Glu Ser Val Val Asp Val Ile Glu Ser Trp Asp Glu Trp Pro Asp
        145                 150                 155

att gaa gaa gat att gcg ttg atc aaa tct gaa gaa gga gaa aaa atg     528
Ile Glu Glu Asp Ile Ala Leu Ile Lys Ser Glu Glu Gly Glu Lys Met

160                 165                 170

gtt ttg gag aat aac ttc ttc gtg gaa acc atg ttg cca tca aaa atc     576
Val Leu Glu Asn Asn Phe Phe Val Glu Thr Met Leu Pro Ser Lys Ile
175                 180                 185                 190

atg aga aag tta gaa cca gaa gaa ttt gca gca tat ctt gaa cca ttc     624
Met Arg Lys Leu Glu Pro Glu Glu Phe Ala Ala Tyr Leu Glu Pro Phe
                195                 200                 205

aaa gag aaa ggt gaa gtt cgt cgt cca aca tta tca tgg cct cgt gaa     672
Lys Glu Lys Gly Glu Val Arg Arg Pro Thr Leu Ser Trp Pro Arg Glu
            210                 215                 220

atc ccg tta gta aaa ggt ggt aaa cct gac gtt gta caa att gtt agg     720
Ile Pro Leu Val Lys Gly Gly Lys Pro Asp Val Val Gln Ile Val Arg
        225                 230                 235

aat tat aat gct tat cta cgt gca agt gat gat tta cca aaa atg ttt     768
Asn Tyr Asn Ala Tyr Leu Arg Ala Ser Asp Asp Leu Pro Lys Met Phe
    240                 245                 250

att gaa tcg gac cca gga ttc ttt tcc aat gct att gtt gaa ggt gcc     816
Ile Glu Ser Asp Pro Gly Phe Phe Ser Asn Ala Ile Val Glu Gly Ala
255                 260                 265                 270

aag aag ttt cct aat act gaa ttt gtc aaa gta aaa ggt ctt cat ttt     864
Lys Lys Phe Pro Asn Thr Glu Phe Val Lys Val Lys Gly Leu His Phe
                275                 280                 285

tcg caa gaa gat gca cct gat gaa atg gga aaa tat atc aaa tcg ttc     912
Ser Gln Glu Asp Ala Pro Asp Glu Met Gly Lys Tyr Ile Lys Ser Phe
            290                 295                 300

gtt gag cga gtt ctc aaa aat gaa caa atg cat atg aaa aag cct gaa     960
Val Glu Arg Val Leu Lys Asn Glu Gln Met His Met Lys Lys Pro Glu
        305                 310                 315

ctc acc gcg acg tct gtc gag aag ttt ctg atc gaa aag ttc gac agc     1008
Leu Thr Ala Thr Ser Val Glu Lys Phe Leu Ile Glu Lys Phe Asp Ser
    320                 325                 330

gtc tcc gac ctg atg cag ctc tcg gag ggc gaa gaa tct cgt gct ttc     1056
Val Ser Asp Leu Met Gln Leu Ser Glu Gly Glu Glu Ser Arg Ala Phe
335                 340                 345                 350

agc ttc gat gta gga ggg cgt gga tat gtc ctg cgg gta aat agc tgc     1104
Ser Phe Asp Val Gly Gly Arg Gly Tyr Val Leu Arg Val Asn Ser Cys
                355                 360                 365

gcc gat ggt ttc tac aaa gat cgt tat gtt tat cgg cac ttt gca tcg     1152
Ala Asp Gly Phe Tyr Lys Asp Arg Tyr Val Tyr Arg His Phe Ala Ser
            370                 375                 380

gcc gcg ctc ccg att ccg gaa gtg ctt gac att ggg gaa ttc agc gag     1200
Ala Ala Leu Pro Ile Pro Glu Val Leu Asp Ile Gly Glu Phe Ser Glu
        385                 390                 395

agc ctg acc tat tgc atc tcc cgc cgt gca cag ggt gtc acg ttg caa     1248
Ser Leu Thr Tyr Cys Ile Ser Arg Arg Ala Gln Gly Val Thr Leu Gln
    400                 405                 410

gac ctg cct gaa acc gaa ctg ccc gct gtt ctg cag ccg gtc gcg gag    1296
Asp Leu Pro Glu Thr Glu Leu Pro Ala Val Leu Gln Pro Val Ala Glu
415                 420                 425                 430

gcc atg gat gcg atc gct gcg gcc gat ctt agc cag acg agc ggg ttc    1344
Ala Met Asp Ala Ile Ala Ala Ala Asp Leu Ser Gln Thr Ser Gly Phe
                435                 440                 445

ggc cca ttc gga ccg caa gga atc ggt caa tac act aca tgg cgt gat    1392
Gly Pro Phe Gly Pro Gln Gly Ile Gly Gln Tyr Thr Thr Trp Arg Asp
            450                 455                 460

ttc ata tgc gcg att gct gat ccc cat gtg tat cac tgg caa act gtg    1440
Phe Ile Cys Ala Ile Ala Asp Pro His Val Tyr His Trp Gln Thr Val
        465                 470                 475

atg gac gac acc gtc agt gcg tcc gtc gcg cag gct ctc gat gag ctg    1488
Met Asp Asp Thr Val Ser Ala Ser Val Ala Gln Ala Leu Asp Glu Leu
    480                 485                 490

atg ctt tgg gcc gag gac tgc ccc gaa gtc cgg cac ctc gtg cac gcg    1536
Met Leu Trp Ala Glu Asp Cys Pro Glu Val Arg His Leu Val His Ala
495                 500                 505                 510

gat ttc ggc tcc aac aat gtc ctg acg gac aat ggc cgc ata aca gcg    1584
Asp Phe Gly Ser Asn Asn Val Leu Thr Asp Asn Gly Arg Ile Thr Ala
                515                 520                 525

gtc att gac tgg agc gag gcg atg ttc ggg gat tcc caa tac gag gtc    1632
Val Ile Asp Trp Ser Glu Ala Met Phe Gly Asp Ser Gln Tyr Glu Val
            530                 535                 540

gcc aac atc ttc ttc tgg agg ccg tgg ttg gct tgt atg gag cag cag    1680
Ala Asn Ile Phe Phe Trp Arg Pro Trp Leu Ala Cys Met Glu Gln Gln
        545                 550                 555

acg cgc tac ttc gag cgg agg cat ccg gag ctt gca gga tcg ccg cgg    1728
Thr Arg Tyr Phe Glu Arg Arg His Pro Glu Leu Ala Gly Ser Pro Arg
    560                 565                 570

ctc cgg gcg tat atg ctc cgc att ggt ctt gac caa ctc tat cag agc    1776
Leu Arg Ala Tyr Met Leu Arg Ile Gly Leu Asp Gln Leu Tyr Gln Ser
575                 580                 585                 590

ttg gtt gac ggc aat ttc gat gat gca gct tgg gcg cag ggt cga tgc    1824
Leu Val Asp Gly Asn Phe Asp Asp Ala Ala Trp Ala Gln Gly Arg Cys
                595                 600                 605

gac gca atc gtc cga tcc gga gcc ggg act gtc ggg cgt aca caa atc    1872
Asp Ala Ile Val Arg Ser Gly Ala Gly Thr Val Gly Arg Thr Gln Ile
            610                 615                 620

gcc cgc aga agc gcg gcc gtc tgg acc gat ggc tgt gta gaa gta ctc    1920
Ala Arg Arg Ser Ala Ala Val Trp Thr Asp Gly Cys Val Glu Val Leu
        625                 630                 635

gcc gat agt gga aac cga cgc ccc agc act cgt ccg agg gca aag gaa    1968
Ala Asp Ser Gly Asn Arg Arg Pro Ser Thr Arg Pro Arg Ala Lys Glu
    640                 645                 650

tagcacacta gtat                                                    1982

<210> 4
<211> 654
<212> PRT
<213> Artificial Sequence
<223> Description of Artificial Sequence: RUHT

<400> 4
Met Thr Ser Lys Val Tyr Asp Pro Glu Gln Arg Lys Arg Met Ile Thr
  1               5                  10                  15

Gly Pro Gln Trp Trp Ala Arg Cys Lys Gln Met Asn Val Leu Asp Ser
             20                  25                  30

Phe Ile Asn Tyr Tyr Asp Ser Glu Lys His Ala Glu Asn Ala Val Ile
         35                  40                  45

Phe Leu His Gly Asn Ala Ala Ser Ser Tyr Leu Trp Arg His Val Val
     50                  55                  60

Pro His Ile Glu Pro Val Ala Arg Cys Ile Ile Pro Asp Leu Ile Gly
 65                  70                  75                  80

Met Gly Lys Ser Gly Lys Ser Gly Asn Gly Ser Tyr Arg Leu Leu Asp
                 85                  90                  95

His Tyr Lys Tyr Leu Thr Ala Trp Phe Glu Leu Leu Asn Leu Pro Lys
            100                 105                 110

Lys Ile Ile Phe Val Gly His Asp Trp Gly Ala Cys Leu Ala Phe His
        115                 120                 125

Tyr Ser Tyr Glu His Gln Asp Lys Ile Lys Ala Ile Val His Ala Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu

                165                 170                 175

Glu Asn Asn Phe Phe Val Glu Thr Met Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Met Phe Ile Glu
                245                 250                 255

Ser Asp Pro Gly Phe Phe Ser Asn Ala Ile Val Glu Gly Ala Lys Lys

            260                 265                 270

Phe Pro Asn Thr Glu Phe Val Lys Val Lys Gly Leu His Phe Ser Gln
        275                 280                 285

Glu Asp Ala Pro Asp Glu Met Gly Lys Tyr Ile Lys Ser Phe Val Glu
    290                 295                 300

Arg Val Leu Lys Asn Glu Gln Met His Met Lys Lys Pro Glu Leu Thr
305                 310                 315                 320

Ala Thr Ser Val Glu Lys Phe Leu Ile Glu Lys Phe Asp Ser Val Ser
                325                 330                 335

Asp Leu Met Gln Leu Ser Glu Gly Glu Glu Ser Arg Ala Phe Ser Phe
            340                 345                 350

Asp Val Gly Gly Arg Gly Tyr Val Leu Arg Val Asn Ser Cys Ala Asp
        355                 360                 365

Gly Phe Tyr Lys Asp Arg Tyr Val Tyr Arg His Phe Ala Ser Ala Ala
    370                 375                 380

Leu Pro Ile Pro Glu Val Leu Asp Ile Gly Glu Phe Ser Glu Ser Leu
385                 390                 395                 400

Thr Tyr Cys Ile Ser Arg Arg Ala Gln Gly Val Thr Leu Gln Asp Leu
                405                 410                 415

Pro Glu Thr Glu Leu Pro Ala Val Leu Gln Pro Val Ala Glu Ala Met
            420                 425                 430

Asp Ala Ile Ala Ala Ala Asp Leu Ser Gln Thr Ser Gly Phe Gly Pro
        435                 440                 445

Phe Gly Pro Gln Gly Ile Gly Gln Tyr Thr Thr Trp Arg Asp Phe Ile
    450                 455                 460

Cys Ala Ile Ala Asp Pro His Val Tyr His Trp Gln Thr Val Met Asp
465                 470                 475                 480

Asp Thr Val Ser Ala Ser Val Ala Gln Ala Leu Asp Glu Leu Met Leu
                485                 490                 495

Trp Ala Glu Asp Cys Pro Glu Val Arg His Leu Val His Ala Asp Phe
            500                 505                 510

Gly Ser Asn Asn Val Leu Thr Asp Asn Gly Arg Ile Thr Ala Val Ile
        515                 520                 525

Asp Trp Ser Glu Ala Met Phe Gly Asp Ser Gln Tyr Glu Val Ala Asn
    530                 535                 540

Ile Phe Phe Trp Arg Pro Trp Leu Ala Cys Met Glu Gln Gln Thr Arg
545                 550                 555                 560

Tyr Phe Glu Arg Arg His Pro Glu Leu Ala Gly Ser Pro Arg Leu Arg
                565                 570                 575

Ala Tyr Met Leu Arg Ile Gly Leu Asp Gln Leu Tyr Gln Ser Leu Val
            580                 585                 590

Asp Gly Asn Phe Asp Asp Ala Ala Trp Ala Gln Gly Arg Cys Asp Ala
        595                 600                 605

Ile Val Arg Ser Gly Ala Gly Thr Val Gly Arg Thr Gln Ile Ala Arg
    610                 615                 620

Arg Ser Ala Ala Val Trp Thr Asp Gly Cys Val Glu Val Leu Ala Asp
625                 630                 635                 640

Ser Gly Asn Arg Arg Pro Ser Thr Arg Pro Arg Ala Lys Glu
                645                 650

<210> 5
<211> 2556
<212> DNA
<213> Artificial Sequence

<220>
<223> Description of Artificial Sequence: LEOK

<220>
<221> CDS
<222> (2)..(2539)

<400> 5
c atg gaa gac gcc aaa aac ata aag aaa ggc ccg gcg cca ttc tat ccg 49
Met Glu Asp Ala Lys Asn Ile Lys Lys Gly Pro Ala Pro Phe Tyr Pro
1               5                   10                  15

ctg gaa gat gga acc gct gga gag caa ctg cat aag gct atg aag aga 97
Leu Glu Asp Gly Thr Ala Gly Glu Gln Leu His Lys Ala Met Lys Arg
            20                  25                  30

tac gcc ctg gtt cct gga aca att gct ttt aca gat gca cat atc gag 145
Tyr Ala Leu Val Pro Gly Thr Ile Ala Phe Thr Asp Ala His Ile Glu
        35                  40                  45

gtg gac atc act tac gct gag tac ttc gaa atg tcc gtt cgg ttg gca 193
Val Asp Ile Thr Tyr Ala Glu Tyr Phe Glu Met Ser Val Arg Leu Ala
    50                  55                  60

gaa gct atg aaa cga tat ggg ctg aat aca aat cac aga atc gtc gta 241
Glu Ala Met Lys Arg Tyr Gly Leu Asn Thr Asn His Arg Ile Val Val
65                  70                  75                  80

tgc agt gaa aac tct ctt caa ttc ttt atg ccg gtg ttg ggc gcg tta 289
Cys Ser Glu Asn Ser Leu Gln Phe Phe Met Pro Val Leu Gly Ala Leu
                85                  90                  95

ttt atc gga gtt gca gtt gcg ccc gcg aac gac att tat aat gaa cgt 337
Phe Ile Gly Val Ala Val Ala Pro Ala Asn Asp Ile Tyr Asn Glu Arg
            100                 105                 110

gaa ttg ctc aac agt atg ggc att tcg cag cct acc gtg gtg ttc gtt 385
Glu Leu Leu Asn Ser Met Gly Ile Ser Gln Pro Thr Val Val Phe Val
        115                 120                 125

tcc aaa aag ggg ttg caa aaa att ttg aac gtg caa aaa aag ctc cca 433

Ser Lys Lys Gly Leu Gln Lys Ile Leu Asn Val Gln Lys Lys Leu Pro
    130                 135                 140

atc atc caa aaa att att atc atg gat tct aaa acg gat tac cag gga     481
Ile Ile Gln Lys Ile Ile Ile Met Asp Ser Lys Thr Asp Tyr Gln Gly
145                 150                 155                 160

ttt cag tcg atg tac acg ttc gtc aca tct cat cta cct ccc ggt ttt     529
Phe Gln Ser Met Tyr Thr Phe Val Thr Ser His Leu Pro Pro Gly Phe
                165                 170                 175

aat gaa tac gat ttt gtg cca gag tcc ttc gat agg gac aag aca att     577
Asn Glu Tyr Asp Phe Val Pro Glu Ser Phe Asp Arg Asp Lys Thr Ile
            180                 185                 190

gca ctg atc atg aac tcc tct gga tct act ggt ctg cct aaa ggt gtc     625
Ala Leu Ile Met Asn Ser Ser Gly Ser Thr Gly Leu Pro Lys Gly Val
        195                 200                 205

gct ctg cct cat aga act gcc tgc gtg aga ttc tcg cat gcc aga gat     673
Ala Leu Pro His Arg Thr Ala Cys Val Arg Phe Ser His Ala Arg Asp
    210                 215                 220

cct att ttt ggc aat caa atc att ccg gat act gcg att tta agt gtt     721
Pro Ile Phe Gly Asn Gln Ile Ile Pro Asp Thr Ala Ile Leu Ser Val
225                 230                 235                 240

gtt cca ttc cat cac ggt ttt gga atg ttt act aca ctc gga tat ttg     769
Val Pro Phe His His Gly Phe Gly Met Phe Thr Thr Leu Gly Tyr Leu
                245                 250                 255

ata tgt gga ttt cga gtc gtc tta atg tat aga ttt gaa gaa gag ctg     817
Ile Cys Gly Phe Arg Val Val Leu Met Tyr Arg Phe Glu Glu Glu Leu
            260                 265                 270

ttt ctg agg agc ctt cag gat tac aag att caa agt gcg ctg ctg gtg     865
Phe Leu Arg Ser Leu Gln Asp Tyr Lys Ile Gln Ser Ala Leu Leu Val
        275                 280                 285

cca acc cta ttc tcc ttc ttc gcc aaa agc act ctg att gac aaa tac     913
Pro Thr Leu Phe Ser Phe Phe Ala Lys Ser Thr Leu Ile Asp Lys Tyr
    290                 295                 300

gat tta tct aat tta cac gaa att gct tct ggt ggc gct ccc ctc tct     961
Asp Leu Ser Asn Leu His Glu Ile Ala Ser Gly Gly Ala Pro Leu Ser
305                 310                 315                 320

aag gaa gtc ggg gaa gcg gtt gcc aag agg ttc cat ctg cca ggt atc     1009
Lys Glu Val Gly Glu Ala Val Ala Lys Arg Phe His Leu Pro Gly Ile
                325                 330                 335

agg caa gga tat ggg ctc act gag act aca tca gct att ctg att aca     1057
Arg Gln Gly Tyr Gly Leu Thr Glu Thr Thr Ser Ala Ile Leu Ile Thr
            340                 345                 350

ccc gag ggg gat gat aaa ccg ggc gcg gtc ggt aaa gtt gtt cca ttt     1105
Pro Glu Gly Asp Asp Lys Pro Gly Ala Val Gly Lys Val Val Pro Phe
        355                 360                 365

ttt gaa gcg aag gtt gtg gat ctg gat acc ggg aaa acg ctg ggc gtt     1153
Phe Glu Ala Lys Val Val Asp Leu Asp Thr Gly Lys Thr Leu Gly Val

370                 375                 380

aat caa aga ggc gaa ctg tgt gtg aga ggt cct atg att atg tcc ggt     1201
Asn Gln Arg Gly Glu Leu Cys Val Arg Gly Pro Met Ile Met Ser Gly
385                 390                 395                 400

tat gta aac aat ccg gaa gcg acc aac gcc ttg att gac aag gat gga     1249
Tyr Val Asn Asn Pro Glu Ala Thr Asn Ala Leu Ile Asp Lys Asp Gly
                405                 410                 415

tgg cta cat tct gga gac ata gct tac tgg gac gaa gac gaa cac ttc     1297
Trp Leu His Ser Gly Asp Ile Ala Tyr Trp Asp Glu Asp Glu His Phe
            420                 425                 430

ttc atc gtt gac cgc ctg aag tct ctg att aag tac aaa ggc tat cag     1345
Phe Ile Val Asp Arg Leu Lys Ser Leu Ile Lys Tyr Lys Gly Tyr Gln
        435                 440                 445

gtg gct ccc gct gaa ttg gaa tcc atc ttg ctc caa cac ccc aac atc     1393
Val Ala Pro Ala Glu Leu Glu Ser Ile Leu Leu Gln His Pro Asn Ile
    450                 455                 460

ttc gac gca ggt gtc gca ggt ctt ccc gac gat gac gcc ggt gaa ctt     1441
Phe Asp Ala Gly Val Ala Gly Leu Pro Asp Asp Asp Ala Gly Glu Leu
465                 470                 475                 480

ccc gcc gcc gtt gtt gtt ttg gag cac gga aag acg atg acg gaa aaa     1489
Pro Ala Ala Val Val Val Leu Glu His Gly Lys Thr Met Thr Glu Lys
                485                 490                 495

gag atc gtg gat tac gtc gcc agt caa gta aca acc gcg aaa aag ttg     1537
Glu Ile Val Asp Tyr Val Ala Ser Gln Val Thr Thr Ala Lys Lys Leu
            500                 505                 510

cgc gga gga gtt gtg ttt gtg gac gaa gta ccg aaa ggt ctt acc gga     1585
Arg Gly Gly Val Val Phe Val Asp Glu Val Pro Lys Gly Leu Thr Gly
        515                 520                 525

aaa ctc gac gca aga aaa atc aga gag atc ctc ata aag gcc aag aag     1633
Lys Leu Asp Ala Arg Lys Ile Arg Glu Ile Leu Ile Lys Ala Lys Lys
    530                 535                 540

ggc gga aag atc gcc gtg atg cat tgt tac att gca caa gat aaa aat     1681
Gly Gly Lys Ile Ala Val Met His Cys Tyr Ile Ala Gln Asp Lys Asn
545                 550                 555                 560

ata tca tca cga aca gta aaa ctg tct gct tac ata aac agt aat aca     1729
Ile Ser Ser Arg Thr Val Lys Leu Ser Ala Tyr Ile Asn Ser Asn Thr
                565                 570                 575

agg ggt gtt atg gga tcg gcc att gaa caa gat gga ttg cac gca ggt     1777
Arg Gly Val Met Gly Ser Ala Ile Glu Gln Asp Gly Leu His Ala Gly
            580                 585                 590

tct ccg gcc gct tgg gtg gag agg cta ttc ggc tat gac tgg gca caa     1825
Ser Pro Ala Ala Trp Val Glu Arg Leu Phe Gly Tyr Asp Trp Ala Gln
        595                 600                 605

cag aca atc ggc tgc tct gat gcc gcc gtg ttc cgg ctg tca gcg cag     1873
Gln Thr Ile Gly Cys Ser Asp Ala Ala Val Phe Arg Leu Ser Ala Gln
    610                 615                 620

ggg cgc ccg gtt ctt ttt gtc aag acc gac ctg tcc ggt gcc ctg aat 1921
Gly Arg Pro Val Leu Phe Val Lys Thr Asp Leu Ser Gly Ala Leu Asn
625                 630                 635                 640

gaa ctg cag gac gag gca gcg cgg cta tcg tgg ctg gcc acg acg ggc 1969
Glu Leu Gln Asp Glu Ala Ala Arg Leu Ser Trp Leu Ala Thr Thr Gly
                645                 650                 655

gtt cct tgc gca gct gtg ctc gac gtt gtc act gaa gcg gga agg gac 2017
Val Pro Cys Ala Ala Val Leu Asp Val Val Thr Glu Ala Gly Arg Asp
            660                 665                 670

tgg ctg cta ttg ggc gaa gtg ccg ggg cag gat ctc ctg tca tct cac 2065
Trp Leu Leu Leu Gly Glu Val Pro Gly Gln Asp Leu Leu Ser Ser His
        675                 680                 685

ctt gct cct gcc gag aaa gta tcc atc atg gct gat gca atg cgg cgg 2113
Leu Ala Pro Ala Glu Lys Val Ser Ile Met Ala Asp Ala Met Arg Arg
    690                 695                 700

ctg cat acg ctt gat ccg gct acc tgc cca ttc gac cac caa gcg aaa 2161
Leu His Thr Leu Asp Pro Ala Thr Cys Pro Phe Asp His Gln Ala Lys
705                 710                 715                 720

cat cgc atc gag cga gca cgt act cgg atg gaa gcc ggt ctt gtc gat 2209
His Arg Ile Glu Arg Ala Arg Thr Arg Met Glu Ala Gly Leu Val Asp
                725                 730                 735

cag gat gat ctg gac gaa gag cat cag ggg ctc gcg cca gcc gaa ctg 2257
Gln Asp Asp Leu Asp Glu Glu His Gln Gly Leu Ala Pro Ala Glu Leu
            740                 745                 750

ttc gcc agg ctc aag gcg cgc atg ccc gac ggc gag gat ctc gtc gtg 2305
Phe Ala Arg Leu Lys Ala Arg Met Pro Asp Gly Glu Asp Leu Val Val
        755                 760                 765

acc cat ggc gat gcc tgc ttg ccg aat atc atg gtg gaa aat ggc cgc 2353
Thr His Gly Asp Ala Cys Leu Pro Asn Ile Met Val Glu Asn Gly Arg
    770                 775                 780

ttt tct gga ttc atc gac tgt ggc cgg ctg ggt gtg gcg gac cgc tat 2401
Phe Ser Gly Phe Ile Asp Cys Gly Arg Leu Gly Val Ala Asp Arg Tyr
785                 790                 795                 800

cag gac ata gcg ttg gct acc cgt gat att gct gaa gag ctt ggc ggc 2449
Gln Asp Ile Ala Leu Ala Thr Arg Asp Ile Ala Glu Glu Leu Gly Gly
                805                 810                 815

gaa tgg gct gac cgc ttc ctc gtg ctt tac ggt atc gcc gct ccc gat 2497
Glu Trp Ala Asp Arg Phe Leu Val Leu Tyr Gly Ile Ala Ala Pro Asp
            820                 825                 830

tcg cag cgc atc gcc ttc tat cgc ctt ctt gac gag ttc ttc 2539
Ser Gln Arg Ile Ala Phe Tyr Arg Leu Leu Asp Glu Phe Phe
        835                 840                 845

tgagtctaga ctagtat 2556

<210> 6

<211> 846
<212> PRT
<213> Artificial Sequence
<223> Description of Artificial Sequence: LEOK

<400> 6
Met Glu Asp Ala Lys Asn Ile Lys Lys Gly Pro Ala Pro Phe Tyr Pro
1               5                   10                  15

Leu Glu Asp Gly Thr Ala Gly Glu Gln Leu His Lys Ala Met Lys Arg
            20                  25                  30

Tyr Ala Leu Val Pro Gly Thr Ile Ala Phe Thr Asp Ala His Ile Glu
        35                  40                  45

Val Asp Ile Thr Tyr Ala Glu Tyr Phe Glu Met Ser Val Arg Leu Ala
    50                  55                  60

Glu Ala Met Lys Arg Tyr Gly Leu Asn Thr Asn His Arg Ile Val Val
65                  70                  75                  80

Cys Ser Glu Asn Ser Leu Gln Phe Phe Met Pro Val Leu Gly Ala Leu
                85                  90                  95

Phe Ile Gly Val Ala Val Ala Pro Ala Asn Asp Ile Tyr Asn Glu Arg
            100                 105                 110

Glu Leu Leu Asn Ser Met Gly Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Leu Pro Lys Gly Val
        195                 200                 205

Ala Leu Pro His Arg Thr Ala Cys Val Arg Phe Ser His Ala Arg Asp
    210                 215                 220

Pro Ile Phe Gly Asn Gln Ile Ile Pro Asp Thr Ala Ile Leu Ser Val
225                 230                 235                 240

Val Pro Phe His His Gly Phe Gly Met Phe Thr Thr Leu Gly Tyr Leu
                245                 250                 255

Ile Cys Gly Phe Arg Val Val Leu Met Tyr Arg Phe Glu Glu Glu Leu
            260                 265                 270

Phe Leu Arg Ser Leu Gln Asp Tyr Lys Ile Gln Ser Ala Leu Leu Val
        275                 280                 285

Pro Thr Leu Phe Ser Phe Phe Ala Lys Ser Thr Leu Ile Asp Lys Tyr

290                 295                 300

Asp Leu Ser Asn Leu His Glu Ile Ala Ser Gly Gly Ala Pro Leu Ser
305                 310                 315                 320

Lys Glu Val Gly Glu Ala Val Ala Lys Arg Phe His Leu Pro Gly Ile
                325                 330                 335

Arg Gln Gly Tyr Gly Leu Thr Glu Thr Thr Ser Ala Ile Leu Ile Thr
            340                 345                 350

Pro Glu Gly Asp Asp Lys Pro Gly Ala Val Gly Lys Val Val Pro Phe
        355                 360                 365

Phe Glu Ala Lys Val Val Asp Leu Asp Thr Gly Lys Thr Leu Gly Val
    370                 375                 380

Asn Gln Arg Gly Glu Leu Cys Val Arg Gly Pro Met Ile Met Ser Gly
385                 390                 395                 400

Tyr Val Asn Asn Pro Glu Ala Thr Asn Ala Leu Ile Asp Lys Asp Gly
                405                 410                 415

Trp Leu His Ser Gly Asp Ile Ala Tyr Trp Asp Glu Asp Glu His Phe
            420                 425                 430

Phe Ile Val Asp Arg Leu Lys Ser Leu Ile Lys Tyr Lys Gly Tyr Gln
        435                 440                 445

Val Ala Pro Ala Glu Leu Glu Ser Ile Leu Leu Gln His Pro Asn Ile
    450                 455                 460

Phe Asp Ala Gly Val Ala Gly Leu Pro Asp Asp Asp Ala Gly Glu Leu
465                 470                 475                 480

Pro Ala Ala Val Val Val Leu Glu His Gly Lys Thr Met Thr Glu Lys
                485                 490                 495

Glu Ile Val Asp Tyr Val Ala Ser Gln Val Thr Thr Ala Lys Lys Leu
            500                 505                 510

Arg Gly Gly Val Val Phe Val Asp Glu Val Pro Lys Gly Leu Thr Gly
        515                 520                 525

Lys Leu Asp Ala Arg Lys Ile Arg Glu Ile Leu Ile Lys Ala Lys Lys
    530                 535                 540

Gly Gly Lys Ile Ala Val Met His Cys Tyr Ile Ala Gln Asp Lys Asn
545                 550                 555                 560

Ile Ser Ser Arg Thr Val Lys Leu Ser Ala Tyr Ile Asn Ser Asn Thr
                565                 570                 575

Arg Gly Val Met Gly Ser Ala Ile Glu Gln Asp Gly Leu His Ala Gly
            580                 585                 590

Ser Pro Ala Ala Trp Val Glu Arg Leu Phe Gly Tyr Asp Trp Ala Gln
        595                 600                 605

Gln Thr Ile Gly Cys Ser Asp Ala Ala Val Phe Arg Leu Ser Ala Gln
    610                 615                 620

Gly Arg Pro Val Leu Phe Val Lys Thr Asp Leu Ser Gly Ala Leu Asn
625                 630                 635                 640

Glu Leu Gln Asp Glu Ala Ala Arg Leu Ser Trp Leu Ala Thr Thr Gly
                645                 650                 655

Val Pro Cys Ala Ala Val Leu Asp Val Val Thr Glu Ala Gly Arg Asp
            660                 665                 670

Trp Leu Leu Leu Gly Glu Val Pro Gly Gln Asp Leu Leu Ser Ser His
        675                 680                 685

Leu Ala Pro Ala Glu Lys Val Ser Ile Met Ala Asp Ala Met Arg Arg
    690                 695                 700

Leu His Thr Leu Asp Pro Ala Thr Cys Pro Phe Asp His Gln Ala Lys
705                 710                 715                 720

His Arg Ile Glu Arg Ala Arg Thr Arg Met Glu Ala Gly Leu Val Asp
                725                 730                 735

Gln Asp Asp Leu Asp Glu Glu His Gln Gly Leu Ala Pro Ala Glu Leu
            740                 745                 750

Phe Ala Arg Leu Lys Ala Arg Met Pro Asp Gly Glu Asp Leu Val Val
        755                 760                 765

Thr His Gly Asp Ala Cys Leu Pro Asn Ile Met Val Glu Asn Gly Arg
    770                 775                 780

Phe Ser Gly Phe Ile Asp Cys Gly Arg Leu Gly Val Ala Asp Arg Tyr
785                 790                 795                 800

Gln Asp Ile Ala Leu Ala Thr Arg Asp Ile Ala Glu Glu Leu Gly Gly
                805                 810                 815

Glu Trp Ala Asp Arg Phe Leu Val Leu Tyr Gly Ile Ala Ala Pro Asp
            820                 825                 830

Ser Gln Arg Ile Ala Phe Tyr Arg Leu Leu Asp Glu Phe Phe
        835                 840                 845

<210> 7
<211> 1846
<212> DNA
<213> Artificial Sequence

<220>
<223> Description of Artificial Sequence: REOK

<220>
<221> CDS
<222> (9)..(1832)

<400> 7
ccatggtc atg act tcg aaa gtt tat gat cca gaa caa agg aaa cgg atg   50
         Met Thr Ser Lys Val Tyr Asp Pro Glu Gln Arg Lys Arg Met
           1               5                  10

ata act ggt ccg cag tgg tgg gcc aga tgt aaa caa atg aat gtt ctt 98
Ile Thr Gly Pro Gln Trp Trp Ala Arg Cys Lys Gln Met Asn Val Leu
15                  20                  25                  30

gat tca ttt att aat tat tat gat tca gaa aaa cat gca gaa aat gct 146
Asp Ser Phe Ile Asn Tyr Tyr Asp Ser Glu Lys His Ala Glu Asn Ala
                35                  40                  45

gtt att ttt tta cat ggt aac gcg gcc tct tct tat tta tgg cga cat 194
Val Ile Phe Leu His Gly Asn Ala Ala Ser Ser Tyr Leu Trp Arg His
            50                  55                  60

gtt gtg cca cat att gag cca gta gcg cgg tgt att ata cca gac ctt 242
Val Val Pro His Ile Glu Pro Val Ala Arg Cys Ile Ile Pro Asp Leu
        65                  70                  75

att ggt atg ggc aaa tca ggc aaa tct ggt aat ggt tct tat agg tta 290
Ile Gly Met Gly Lys Ser Gly Lys Ser Gly Asn Gly Ser Tyr Arg Leu
    80                  85                  90

ctt gat cat tac aaa tat ctt act gca tgg ttt gaa ctt ctt aat tta 338
Leu Asp His Tyr Lys Tyr Leu Thr Ala Trp Phe Glu Leu Leu Asn Leu
95                  100                 105                 110

cca aag aag atc att ttt gtc ggc cat gat tgg ggt gct tgt ttg gca 386

Pro Lys Lys Ile Ile Phe Val Gly His Asp Trp Gly Ala Cys Leu Ala
                115                 120                 125

ttt cat tat agc tat gag cat caa gat aag atc aaa gca ata gtt cac 434
Phe His Tyr Ser Tyr Glu His Gln Asp Lys Ile Lys Ala Ile Val His
            130                 135                 140

gct gaa agt gta gta gat gtg att gaa tca tgg gat gaa tgg cct gat 482
Ala Glu Ser Val Val Asp Val Ile Glu Ser Trp Asp Glu Trp Pro Asp
        145                 150                 155

att gaa gaa gat att gcg ttg atc aaa tct gaa gaa gga gaa aaa atg 530
Ile Glu Glu Asp Ile Ala Leu Ile Lys Ser Glu Glu Gly Glu Lys Met
    160                 165                 170

gtt ttg gag aat aac ttc ttc gtg gaa acc atg ttg cca tca aaa atc 578
Val Leu Glu Asn Asn Phe Phe Val Glu Thr Met Leu Pro Ser Lys Ile
175                 180                 185                 190

atg aga aag tta gaa cca gaa gaa ttt gca gca tat ctt gaa cca ttc 626
Met Arg Lys Leu Glu Pro Glu Glu Phe Ala Ala Tyr Leu Glu Pro Phe
                195                 200                 205

aaa gag aaa ggt gaa gtt cgt cgt cca aca tta tca tgg cct cgt gaa 674
Lys Glu Lys Gly Glu Val Arg Arg Pro Thr Leu Ser Trp Pro Arg Glu
            210                 215                 220

atc ccg tta gta aaa ggt ggt aaa cct gac gtt gta caa att gtt agg 722
Ile Pro Leu Val Lys Gly Gly Lys Pro Asp Val Val Gln Ile Val Arg
        225                 230                 235

aat tat aat gct tat cta cgt gca agt gat gat tta cca aaa atg ttt 770
Asn Tyr Asn Ala Tyr Leu Arg Ala Ser Asp Asp Leu Pro Lys Met Phe
    240                 245                 250

att gaa tcg gac cca gga ttc ttt tcc aat gct att gtt gaa ggt gcc 818
Ile Glu Ser Asp Pro Gly Phe Phe Ser Asn Ala Ile Val Glu Gly Ala
255                 260                 265                 270

aag aag ttt cct aat act gaa ttt gtc aaa gta aaa ggt ctt cat ttt 866
Lys Lys Phe Pro Asn Thr Glu Phe Val Lys Val Lys Gly Leu His Phe
                275                 280                 285

tcg caa gaa gat gca cct gat gaa atg gga aaa tat atc aaa tcg ttc 914
Ser Gln Glu Asp Ala Pro Asp Glu Met Gly Lys Tyr Ile Lys Ser Phe
            290                 295                 300

gtt gag cga gtt ctc aaa aat gaa caa atg cat tgt tac att gca caa 962
Val Glu Arg Val Leu Lys Asn Glu Gln Met His Cys Tyr Ile Ala Gln
        305                 310                 315

gat aaa aat ata tca tca cga aca gta aaa ctg tct gct tac ata aac 1010
Asp Lys Asn Ile Ser Ser Arg Thr Val Lys Leu Ser Ala Tyr Ile Asn
    320                 325                 330

agt aat aca agg ggt gtt atg gga tcg gcc att gaa caa gat gga ttg 1058
Ser Asn Thr Arg Gly Val Met Gly Ser Ala Ile Glu Gln Asp Gly Leu
335                 340                 345                 350

cac gca ggt tct ccg gcc gct tgg gtg gag agg cta ttc ggc tat gac 1106
His Ala Gly Ser Pro Ala Ala Trp Val Glu Arg Leu Phe Gly Tyr Asp
                355                 360                 365

tgg gca caa cag aca atc ggc tgc tct gat gcc gcc gtg ttc cgg ctg 1154
Trp Ala Gln Gln Thr Ile Gly Cys Ser Asp Ala Ala Val Phe Arg Leu
            370                 375                 380

tca gcg cag ggg cgc ccg gtt ctt ttt gtc aag acc gac ctg tcc ggt 1202
Ser Ala Gln Gly Arg Pro Val Leu Phe Val Lys Thr Asp Leu Ser Gly
        385                 390                 395

gcc ctg aat gaa ctg cag gac gag gca gcg cgg cta tcg tgg ctg gcc 1250
Ala Leu Asn Glu Leu Gln Asp Glu Ala Ala Arg Leu Ser Trp Leu Ala
    400                 405                 410

acg acg ggc gtt cct tgc gca gct gtg ctc gac gtt gtc act gaa gcg 1298
Thr Thr Gly Val Pro Cys Ala Ala Val Leu Asp Val Val Thr Glu Ala
415                 420                 425                 430

gga agg gac tgg ctg cta ttg ggc gaa gtg ccg ggg cag gat ctc ctg 1346
Gly Arg Asp Trp Leu Leu Leu Gly Glu Val Pro Gly Gln Asp Leu Leu
                435                 440                 445

tca tct cac ctt gct cct gcc gag aaa gta tcc atc atg gct gat gca 1394
Ser Ser His Leu Ala Pro Ala Glu Lys Val Ser Ile Met Ala Asp Ala
            450                 455                 460

atg cgg cgg ctg cat acg ctt gat ccg gct acc tgc cca ttc gac cac 1442
Met Arg Arg Leu His Thr Leu Asp Pro Ala Thr Cys Pro Phe Asp His
        465                 470                 475

caa gcg aaa cat cgc atc gag cga gca cgt act cgg atg gaa gcc ggt 1490
Gln Ala Lys His Arg Ile Glu Arg Ala Arg Thr Arg Met Glu Ala Gly
    480                 485                 490

ctt gtc gat cag gat gat ctg gac gaa gag cat cag ggg ctc gcg cca    1538
Leu Val Asp Gln Asp Asp Leu Asp Glu Glu His Gln Gly Leu Ala Pro
495                 500                 505                 510

gcc gaa ctg ttc gcc agg ctc aag gcg cgc atg ccc gac ggc gag gat    1586
Ala Glu Leu Phe Ala Arg Leu Lys Ala Arg Met Pro Asp Gly Glu Asp
                515                 520                 525

ctc gtc gtg acc cat ggc gat gcc tgc ttg ccg aat atc atg gtg gaa    1634
Leu Val Val Thr His Gly Asp Ala Cys Leu Pro Asn Ile Met Val Glu
            530                 535                 540

aat ggc cgc ttt tct gga ttc atc gac tgt ggc cgg ctg ggt gtg gcg    1682
Asn Gly Arg Phe Ser Gly Phe Ile Asp Cys Gly Arg Leu Gly Val Ala
        545                 550                 555

gac cgc tat cag gac ata gcg ttg gct acc cgt gat att gct gaa gag    1730
Asp Arg Tyr Gln Asp Ile Ala Leu Ala Thr Arg Asp Ile Ala Glu Glu
    560                 565                 570

ctt ggc ggc gaa tgg gct gac cgc ttc ctc gtg ctt tac ggt atc gcc    1778
Leu Gly Gly Glu Trp Ala Asp Arg Phe Leu Val Leu Tyr Gly Ile Ala
575                 580                 585                 590

gct ccc gat tcg cag cgc atc gcc ttc tat cgc ctt ctt gac gag ttc    1826
Ala Pro Asp Ser Gln Arg Ile Ala Phe Tyr Arg Leu Leu Asp Glu Phe
                595                 600                 605

ttc tga gtctagacta gtat                                            1846
Phe


<210> 8
<211> 607
<212> PRT
<213> Artificial Sequence
<223> Description of Artificial Sequence: REOK

<400> 8
Met Thr Ser Lys Val Tyr Asp Pro Glu Gln Arg Lys Arg Met Ile Thr
  1               5                  10                  15

Gly Pro Gln Trp Trp Ala Arg Cys Lys Gln Met Asn Val Leu Asp Ser
             20                  25                  30

Phe Ile Asn Tyr Tyr Asp Ser Glu Lys His Ala Glu Asn Ala Val Ile
         35                  40                  45

Phe Leu His Gly Asn Ala Ala Ser Ser Tyr Leu Trp Arg His Val Val
     50                  55                  60

Pro His Ile Glu Pro Val Ala Arg Cys Ile Ile Pro Asp Leu Ile Gly
 65                  70                  75                  80

Met Gly Lys Ser Gly Lys Ser Gly Asn Gly Ser Tyr Arg Leu Leu Asp
                 85                  90                  95

His Tyr Lys Tyr Leu Thr Ala Trp Phe Glu Leu Leu Asn Leu Pro Lys
            100                 105                 110

Lys Ile Ile Phe Val Gly His Asp Trp Gly Ala Cys Leu Ala Phe His

        115                 120                 125

Tyr Ser Tyr Glu His Gln Asp Lys Ile Lys Ala Ile Val His Ala Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu
                165                 170                 175

Glu Asn Asn Phe Phe Val Glu Thr Met Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Met Phe Ile Glu
                245                 250                 255

Ser Asp Pro Gly Phe Phe Ser Asn Ala Ile Val Glu Gly Ala Lys Lys
            260                 265                 270

Phe Pro Asn Thr Glu Phe Val Lys Val Lys Gly Leu His Phe Ser Gln
        275                 280                 285

Glu Asp Ala Pro Asp Glu Met Gly Lys Tyr Ile Lys Ser Phe Val Glu
    290                 295                 300

Arg Val Leu Lys Asn Glu Gln Met His Cys Tyr Ile Ala Gln Asp Lys
305                 310                 315                 320

Asn Ile Ser Ser Arg Thr Val Lys Leu Ser Ala Tyr Ile Asn Ser Asn
                325                 330                 335

Thr Arg Gly Val Met Gly Ser Ala Ile Glu Gln Asp Gly Leu His Ala
            340                 345                 350

Gly Ser Pro Ala Ala Trp Val Glu Arg Leu Phe Gly Tyr Asp Trp Ala
        355                 360                 365

Gln Gln Thr Ile Gly Cys Ser Asp Ala Ala Val Phe Arg Leu Ser Ala
    370                 375                 380

Gln Gly Arg Pro Val Leu Phe Val Lys Thr Asp Leu Ser Gly Ala Leu
385                 390                 395                 400

Asn Glu Leu Gln Asp Glu Ala Ala Arg Leu Ser Trp Leu Ala Thr Thr
                405                 410                 415

Gly Val Pro Cys Ala Ala Val Leu Asp Val Val Thr Glu Ala Gly Arg
            420                 425                 430

Asp Trp Leu Leu Leu Gly Glu Val Pro Gly Gln Asp Leu Leu Ser Ser
        435                 440                 445

His Leu Ala Pro Ala Glu Lys Val Ser Ile Met Ala Asp Ala Met Arg
    450                 455                 460

Arg Leu His Thr Leu Asp Pro Ala Thr Cys Pro Phe Asp His Gln Ala
465                 470                 475                 480

Lys His Arg Ile Glu Arg Ala Arg Thr Arg Met Glu Ala Gly Leu Val
                485                 490                 495

Asp Gln Asp Asp Leu Asp Glu Glu His Gln Gly Leu Ala Pro Ala Glu
            500                 505                 510

Leu Phe Ala Arg Leu Lys Ala Arg Met Pro Asp Gly Glu Asp Leu Val
        515                 520                 525

Val Thr His Gly Asp Ala Cys Leu Pro Asn Ile Met Val Glu Asn Gly
    530                 535                 540

Arg Phe Ser Gly Phe Ile Asp Cys Gly Arg Leu Gly Val Ala Asp Arg
545                 550                 555                 560

Tyr Gln Asp Ile Ala Leu Ala Thr Arg Asp Ile Ala Glu Glu Leu Gly
                565                 570                 575

Gly Glu Trp Ala Asp Arg Phe Leu Val Leu Tyr Gly Ile Ala Ala Pro
            580                 585                 590

Asp Ser Gln Arg Ile Ala Phe Tyr Arg Leu Leu Asp Glu Phe Phe
        595                 600                 605

<210> 9
<211> 85
<212> DNA
<213> Artificial Sequence

<220>
<223> Description of Artificial Sequence: Primer

<400> 9
tggagcaaga tcagatgtaa atgggaatcc aagatagtgg agaagaagta aagggtcgac 60
ccctgccctg aaccgacgac cgggt                                       85

<210> 10
<211> 84
<212> DNA
<213> Artificial Sequence

<220>
<223> Description of Artificial Sequence: Primer

<400> 10
ctgtactatt caaacctcac agccatgttt gcaaacactg actttgtggg ggacgcgtcg 60
aataaatacc tgtgacggaa gatg                                        84